(19) Europäisches Patentamt
European Patent Office
Office européen des brevets

(11) Veröffentlichungsnummer : **0 069 968**
**B1**

(12) # EUROPÄISCHE PATENTSCHRIFT

(45) Veröffentlichungstag der Patentschrift : **21.11.85**

(51) Int. Cl.⁴ : **C 07 F 9/10**, C 07 F 9/09, A 61 K 31/685

(21) Anmeldenummer : **82106024.1**

(22) Anmeldetag : **06.07.82**

(54) **Phospholipide, Verfahren zu deren Herstellung und diese Verbindungen enthaltende Arzneimittel.**

(30) Priorität : **11.07.81 DE 3127503**

(43) Veröffentlichungstag der Anmeldung : **19.01.83 Patentblatt 83/03**

(45) Bekanntmachung des Hinweises auf die Patenterteilung : **21.11.85 Patentblatt 85/47**

(84) Benannte Vertragsstaaten :
**AT BE CH DE FR GB IT LI LU NL SE**

(56) Entgegenhaltungen :
**EP-A- 0 040 039**
**EP-A- 0 050 327**
**EP-A- 0 061 872**
**DE-A- 2 619 686**
**DE-A- 2 642 661**
**CHEMICAL ABSTRACTS, Band 93, Nr. 3, 21. Juli 1980, Seite 653, Nr. 25917n, Columbus, Ohio, USA**
**CA vol. 83, 1975, Referat 175990V**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Bosies, Elmar, Dr. rer. nat.**
**Delpstrasse 11**
**D-6940 Weinheim (DE)**
Erfinder : **Gall, Rudi, Dr. phil.**
**Ulmenstrasse 1**
**D-6945 Hirschberg 1 (DE)**
Erfinder : **Weimann, Günter, Dr. rer. nat.**
**Panorama-Strasse 6**
**D-6940 Weinheim-Lützelsachsen (DE)**
Erfinder : **Bicker, Uwe, Dr. rer. nat.**
**Hirschstrasse 59**
**D-6143 Lorsch (DE)**
Erfinder : **Pahlke, Wulf, Dr.**
**Ziegelgasse 33**
**D-6800 Mannheim 31 (DE)**

**EP 0 069 968 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Phospholipide, deren pharmakologisch unbedenklichen Salze, Verfahren zu deren Herstellung, sowie Arzneimittel, die diese Substanzen enthalten.

In der DE-AS-20 09 341 wird der 3-Octadecyloxypropanol-(1)-phosphorsäure-mono-cholinester als immunologisches Adjuvans, in der DE-AS-20 09 342 dessen 2-Hydroxyderivat als Mittel zur Steigerung der natürlichen Resistenz des Organismus und in der DE-OS 26 19 686 dessen 2-Methoxyderivat als Antitumormittel beansprucht. Schließlich ist in der DE-OS 26 19 715 das Dodecyloxypropylphosphorylcholin als « Tumorantigen » beschrieben.

In der japanischen Offenlegungsschrift JP-A-5002-636 sind allgemein einige Glycero-phosphorylcholine mit Urethan- bzw. Thiourethan-Gruppierungen beschrieben. In der DE-OS 27 17 597 werden Phospholipide beschrieben, deren Alkylkette in 3-Stellung des Glycerins durch Halogen oder Phenyl substituiert sein kann.

In EP-A 1-0.040 039 und EP-A 1-0.061.872 sowie der DE-OS 26 42 661 sind Ethylenglykol-phosphorylcholin-Derivate mit einer Hemmwirkung auf die Vermehrung von Tumoren beschrieben.

Es wurde gefunden, daß die neuen Phospholipide der vorliegenden Anmeldung hervorragend cancerostatisch wirken, aber im Gegensatz zu den obengenannten Phospholipiden keine Thrombozytenaggregation auslösen.

Gegenstand der vorliegenden Erfindung sind nun neue Verbindungen der allgemeinen Formel (I),

$$R_1-X-R_2-Y-R_3-Z-\underset{O^{\ominus}}{\overset{O}{\overset{\|}{P}}}-O-R_4-\overset{\oplus}{N}(R_5)_3 \qquad (I)$$

in der

X = Valenzstrich, Sauerstoff, Schwefel, die Sulfonyl-, die Sulfinylgruppe, ein Phenylen-, ein $C_3$-$C_8$ Cycloalkylenrest, die —CONH—, —NHCO—, —NHCONH— oder Carbonylgruppe,

$R_1$ = Wasserstoff oder ein geradkettiger oder verzweigter, gesättigter oder ungesättigter Alkylrest mit 1-18 bzw. 2-18 Kohlenstoffatomen, der gegebenenfalls ein oder mehrfach durch Halogen, eine Alkoxy-, Alkylmercapto-, Alkansulfinyl-, Alkansulfonyl-, Carbalkoxygruppe oder den Phenylrest substituiert ist,

$R_2$ = eine geradkettige oder verzweigte, gesättigte oder ungesättigte Alkylenkette mit 1-18 bzw. 2-18 Kohlenstoffatomen, die gegebenenfalls ein- oder mehrfach durch Halogen, eine Alkoxy-, Alkylmercapto-, Alkansulfinyl-, Alkansulfonyl-, Carbalkoxygruppe oder den Phenylrest substituiert ist,

Y = Sauerstoff, O—CO—O, O—CO—NH, O—CS—NH,

$R_3$ = $—CH_2—CH_2—CH_2$, die auch Teil eines Cyclopentan-, Cyclohexan- und Cycloheptan-Ringsystems sein kann und die gegebenenfalls ein oder mehrfach durch Hydroxy, Halogen, eine Alkylmercapto-, Alkansulfinyl-, Alkansulfonylgruppe, die Nitril-, Alkoxycarbonyl- oder eine gegebenenfalls durch Alkylgruppen substituierte Carbamoylgruppe, einen Cyclopentyl-, Cyclohexyl- oder Cycloheptyl-Rest, einen gegebenenfalls substituierten Phenylrest oder eine Alkoxygruppe substituiert ist, die ihrerseits gegebenenfalls durch Phenyl, Hydroxy, eine Alkoxy-, Alkylmercapto-, Alkansulfinyl-, Alkansulfonyl-, eine gegebenenfalls acylierte Aminogruppe, einen Alkoxycarbonyl-, die Nitril- oder einen gegebenenfalls durch Alkylgruppen substituierten Carbamoylrest substituiert ist,

Z = Sauerstoff oder Schwefel,

$R_4$ = eine geradkettige oder verzweigte Alkylenkette mit 2-5 Kohlenstoffatomen,

$R_5$ = Wasserstoff oder eine niedere Alkylgruppe bedeuten,

wobei für den Fall, daß die Gruppe $R_1—X—R_2$ eine unsubstituierte geradkettige oder verzweigte, gesättigte oder ungesättigte Alkylkette mit 1-18 bzw. 2-18 Kohlenstoffatomen und Z Sauerstoff darstellen,

$R_3$ = nicht gleich eine Propylenkette sein darf, die jeweils gegebenenfalls durch Hydroxy, Alkoxy oder Benzyloxy substituiert ist,

und wobei für den Fall, daß die Gruppe $R_1—X—R_2$ eine durch Halogen oder Phenyl substituierte Alkylkette mit 1-18 Kohlenstoffatomen darstellt und Y und Z = Sauerstoff bedeuten, $R_3$ nicht gleich eine Propylen- oder 2-Hydroxypropylenkette sein darf,

sowie deren pharmakologisch unbedenklichen Salze.

Ferner sind Gegenstand der Erfindung Verbindungen der Formel Ia

$$C_{18}H_{37}-O-R_{3a}-O-\underset{O^{\ominus}}{\overset{O}{\overset{\|}{P}}}-O-CH_2CH_2\overset{\oplus}{N}(CH_3)_3 \qquad (Ia),$$

in der

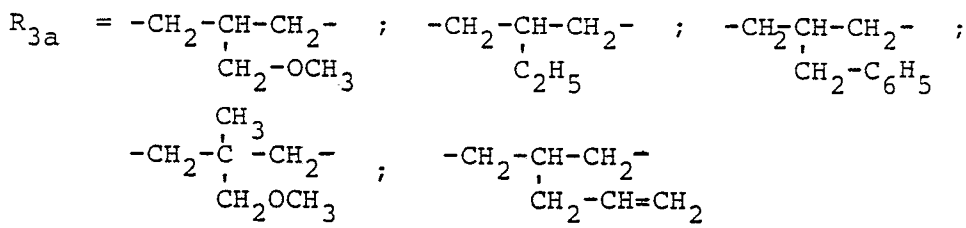

bedeutet,
sowie deren pharmakologisch unbedenkliche Salze, der Formel Ib

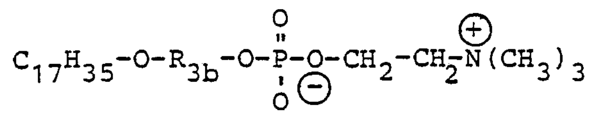
(Ib),

in der

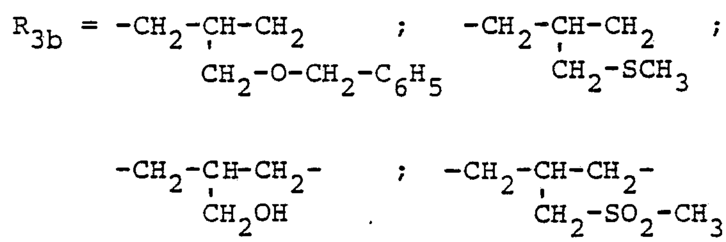

bedeutet,
sowie deren pharmakologisch unbedenkliche Salze, der Formel Ic

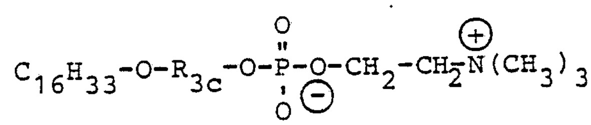
(Ic)

in der

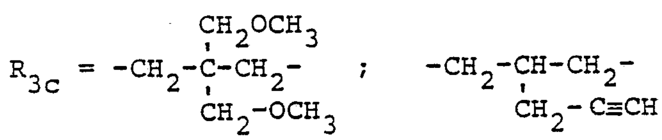

bedeutet,
sowie deren pharmakologisch unbedenkliche Salze und die Verbindung
[2-Methyl-1-(3-tetradecyloxy-propoxy)-3-propyl]-(2-trimethylammonium-ethyl)-phosphat
sowie deren pharmakologisch unbedenkliche Salze.

Alkyl bedeutet in dem Substituenten $R_5$ ein Kohlenwasserstoffrest mit 1-6 Kohlenstoffatomen, insbesondere der Methyl- oder Ethylrest.

Alkoxy, Alkoxycarbonyl, Alkylmercapto, Alkansulfinyl und Alkansulfonyl bedeuten in allen Fällen in der Regel Reste mit 1-6 Kohlenstoffatomen, können aber auch Reste mit bis zu 20 Kohlenstoffatomen sein, z. B. Octadecyloxy, Tetradecyloxy, Octyloxy.

Eine Acylamino-Gruppe bedeutet insbesondere eine Amino-Gruppe, die durch Acetyl- oder Methansulfonyl substituiert ist.

Unter Cycloalkylen-Rest der Gruppe X sind Rest mit 3-8 Kohlenstoffatomen zu verstehen, insbesondere der Cyclopropyl-, Cyclopentyl- und Cyclohexyl-Rest.

Halogen bedeutet Fluor, Chlor, Brom und Jod, vorzugsweise jedoch Fluor und Chlor.

Die Gruppe $R_1$—X—$R_2$ bedeutet für den Fall, daß X einen Valenzstrich darstellt, eine Alkylkette mit 1-18 Kohlenstoffatomen, die geradkettig oder verzweigt, gesättigt oder ungesättigt sein kann. Die ungesättigte Gruppe kann bis zu 4 Doppelbindungen enthalten, vorzugsweise jedoch 1 oder 2 Doppelbindungen.

Die Gruppe $R_4$ bedeutet bevorzugt die $—CH_2—CH_2$-Gruppe.

Der bei der Gruppe $R_3$ aufgeführte Phenylrest kann ein- oder mehrfach durch Alkyl, Alkoxy oder Halogen substituiert sein.

Weiter sind Gegenstand der Erfindung Verfahren zur Herstellung der Verbindungen der allgemeinen Formel I

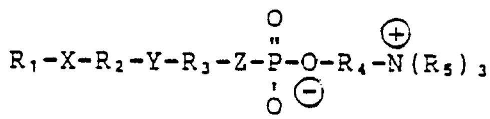

(I)

in der $R_1$ bis $R_5$, X, Y und Z die oben angegebene Bedeutung haben, indem man eine Verbindung der allgemeinen Formel II

$$R_1—X—R_2—Y—R_3—ZH \quad (II),$$

in der $R_1$, $R_2$, $R_3$, X, Y und Z die oben angegebene Bedeutung haben, entweder

a) mit einer Verbindung der allgemeinen Formel III oder IIIa

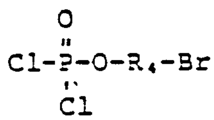

(III)

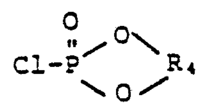

(IIIa)

in denen $R_4$ die oben genannte Bedeutung hat, in Gegenwart eines säurebindenden Mittels umsetzt, das Reaktionsprodukt bei Verwendung einer Verbindung der Formel III selektiv hydrolysiert und das verbleibende Bromatom gegen eine gegebenenfalls alkylierte Ammoniumgruppe austauscht, bei Verwendung einer Verbindung der Formel IIIa direkt mit gegebenenfalls alkyliertem Ammoniak behandelt oder

b) in eine Verbindung der allgemeinen Formel IV überführt

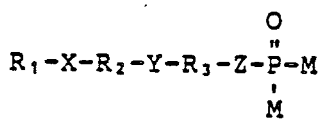

(IV)

in der $R_1$, $R_2$, $R_3$, X, Y und Z die oben angegebene Bedeutungen haben und M Hydroxy, Chlor, Brom oder Alkylmercapto bedeuten, und diese mit einer Verbindung der allgemeinen Formel V

$$HO—R_4—T \quad (V),$$

in der $R_4$ die oben angegebene Bedeutung hat und T Chlor oder Brom oder die Gruppe $N(R_5)_3^+$ $Hal^-$ darstellt, wobei $R_5$ die oben angegebene Bedeutung hat und $Hal^-$ Chlorid, Bromid oder Jodid sein soll, umsetzt- und für den Fall, daß T Chlor oder Brom ist, das so erhaltene Zwischenprodukt anschließend mit einem Amin $N(R_5)_3$ quaternisiert, oder

c) mit einer Verbindung der allgemeinen Formel VI

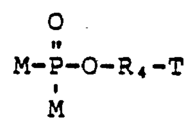

(VI)

in der M, $R_4$ und T die oben angegebenen Bedeutungen haben in Gegenwart eines säure- oder wasserbindenden Mittels umsetzt, in das innere Salz überführt und gegebenenfalls oxidiert.

Die Verbindungen der Formeln Ia, Ib, Ic sowie [2-Methyl-1-(3-tetradecyloxy-propoxy)-3-propyl]-(2-trimethylammonium-ethyl)-phosphat werden in entsprechender Weise hergestellt.

Alle beschriebenen Verfahren erfolgen in an sich bekannter Weise.

Das Verfahren a) wird in der Regel so durchgeführt, daß ein Alkohol oder Thiol der allgemeinen Formel II mit einem Bromalkylphosphorsäuremonoester-dichlorid der allgemeinen Formel II in Gegenwart eines säurebindenden Mittels, wie z. B. Triethylamin in einem absoluten, inerten organischen Lösungsmittel, wie z. B. chlorierter Kohlenwasserstoff oder Toluol, bei Temperaturen um den Gefrierpunkt bis Raumtemperatur reagiert. Die selektive Hydrolyse des erhaltenen Phosphorsäurediester-monochlorids wird direkt in zweiphasiger Mischung durch Zugabe einer wässrigen Kaliumchloridlösung bei 0 °C bis 50 °C erreicht. Zur Substitution des verbliebenen Bromatoms durch eine gegebenenfalls alkylierte Ammoniumgruppe löst man den Ammoniak oder das Alkylamin in einem Medium, das sowohl den Phosphorsäurediester als auch Ammoniak oder das Amin genügend gut löst, wozu sich besonders Mischungen aus Acetonitril oder niederen Alkoholen mit chlorierten Kohlenwasserstoffen eignen, und vervollständigt die Reaktion bei einer Temperatur von 20 °C bis 70 °C.

Man kann auch stufenweise vorgehen, also zuerst eine Alkylammoniumgruppe einführen und mit Alkylhalogenid anschließend zum Di- oder Trialkylammoniumalkylester umsetzen.

Wird ein Alkohol oder Thiol der allgemeinen Formel II mit einem Phosphoresterchlorid der

allgemeinen Formel III a umgesetzt, geschieht dies unter den gleichen Reaktionsbedingungen wie vorher angegeben. Die Umsetzung mit gegebenenfalls alkyliertem Ammoniak geschieht bevorzugt in Acetonitril als Lösungsmittel bei 50-100 °C in einem Druckgefäß.

Die Entfernung der restlichen Halogenidionen erfolgt vorzugsweise in niederen Alkoholen mittels Silberacetat oder Silberoxid.

Sämtliche Zwischenstufen sowie Endprodukte lassen sich säulenchromatographisch mit üblichen Elutionsmitteln, wie z. B. Ether, Ligroin, chlorierten Kohlenwasserstoffen, niederen Alkoholen oder Mischungen derselben, an Kieselgel reinigen, wobei im Falle des betainartigen Endprodukts zweckmäßig etwas Wasser zugesetzt wird.

Bei Verfahren b) wird, falls M Chlor oder Brom bedeutet, die Umsetzung einer Verbindung der allgemeinen Formel II zum Phosphoresterdihalogenid der allgemeinen Formel IV mit Phosphoroxyhalogenid in inerten, wasserfreien Lösungsmitteln wie z. B. halogenierten Kohlenwasserstoffen in Gegenwart von Säureacceptoren, vorzugsweise Pyridin oder Chinolin, vorgenommen. Die Reaktionstemperaturen liegen zwischen 0 °C und 40 °C. Das Produkt kann isoliert werden oder ohne Isolierung mit einem Alkohol der allgemeinen Formel V unter Zusatz von weiterem Pyridin oder Chinolin bei Temperaturen von 0 °C bis 40 °C umgesetzt werden. Als Lösungsmittel hierfür verwendet man bevorzugt halogenierte Kohlenwasserstoffe sowie Acetonitril oder Trichloracetonitril.

Für den Fall, daß M Hydroxy bedeutet, kann die Verbindung der allgemeinen Formel IV nach allgemein üblichen Verfahren, wie z. B. durch Hydrolyse des entsprechenden Phosphoresterdihalogenids oder durch Hydrogenolyse des entsprechenden Phosphoresterdiphenylesters hergestellt werden. Die weitere Umsetzung mit Alkoholen der allgemeinen Formel V geschieht in Gegenwart von Sulfonsäurehalogeniden, wie z. B. p-Toluolsulfochlorid oder Triisopropylbenzolsulfochlorid. Als Lösungsmittel verwendet man Dimethylformamid mit einem Zusatz an Pyridin oder Pyridin allein. Die Reaktionstemperaturen liegen in der Regel bei 0 °C bis 40 °C. Als aktivierendes und wasserentziehendes Mittel kann auch sehr gut Dicyclohexylcarbodiimid eingesetzt werden.

Für den Fall, daß M eine Alkylmercaptogruppe bedeutet, kann die Verbindung der allgemeinen Formel IV durch Umsetzung einer Verbindung der allgemeinen Formel II mit einem Thiophosphorsäurealkylester-di-alkalisalz erhalten werden. Als wasserabspaltendes Mittel setzt man bevorzugt Dicyclohexylcarbodiimid in Pyridin ein. Die Reaktion wird bei Zimmertemperatur durchgeführt. Dieser so erhaltene Alkylthiophosphorsäureester wird mit einem Alkohol der allgemeinen Formel V in Gegenwart von Jod umgesetzt.

In allen Fällen, bei denen T in einer Substanz der allgemeinen Formel V Chlor oder Brom bedeutet, wird das so erhaltene Zwischenprodukt unter den bei Verfahren a) angegebenen Bedingungen zum gewünschten Endprodukt umgesetzt.

Die Substanzen der allgemeinen Formel (VI) bei Verfahren c) können für den Fall, daß M Hydroxy bedeutet, mit allgemein üblichen Halogenierungsmitteln, wie z. B. Phosphorpentachlorid, in Gegenwart eines Säureacceptors, wie z. B. Pyridin zu Substanzen der allgemeinen Formel (VI) mit M = Chlor oder Brom umgesetzt werden, die dann isoliert werden können oder ohne Isolierung mit Verbindungen der allgemeinen Formel (II) zur Reaktion gebracht werden. Als Säureacceptoren werden hier in der Regel ebenfalls stickstoffhaltige Basen wie Pyridin, Chinolin oder Triethylamin eingesetzt. Die bevorzugten Lösungsmittel sind wasserfreie halogenierte Kohlenwasserstoffe oder auch Toluol. Für den Fall, daß M Hydroxy oder Alkylmercapto darstellt, werden die Reaktionen bei den unter Verfahren b) beschriebenen Bedingunger durchgeführt.

Die als Ausgangsmaterial eingesetzten Alkohole oder Thiole der allgemeinen Formel II sind ebenfalls neu. Die Alkohole der allgemeinen Formel II lassen sich z. B. durch Reduktion der entsprechenden Carbonsäureester mit komplexen Hybriden wie z. B. Lithiumaluminiumhydrid herstellen. Man kann jedoch auch ein Diol der allgemeinen Formel VII

$$HO—R_3—OH \qquad (VII),$$

wobei $R_3$ die oben angegebene Bedeutung hat, mit äquimolaren Mengen eines entsprechenden Halogenids, Sulfonats, Chlorameisensäureester, Isocyanats oder Isothiocyanats zum gewünschten Alkohol der allgemeinen Formel II umsetzen.

Die Thiole der allgemeinen Formel II lassen sich aus den Alkoholen der allgemeinen Formel II nach an sich bekannten Methoden synthetisieren, indem man z. B. aus dem Alkohol der allgemeinen Formel II ein Halogenid oder Sulfonat herstellt, dieses mit Thioharnstoff reagieren läßt und das entstandene Isothiuroniumsalz mit Alkali spaltet. Durch Ansäuern erhält man dann das gewünschte Thiol der allgemeinen Formel II.

Gegenstand der Erfindung sind ferner sämtliche stereoisomeren Verbindungen der Formel (I), die z. B. aufgrund asymmetrischer Kohlenstoffatome, der Sulfoxidgruppe oder aufgrund von cis-trans-Isomerie anfallen. Eine Trennung der in einem Gemisch anfallenden Produkte geschieht nach an sich bekannten Verfahren.

Die pharmakologisch verträglichen Salze erhält man in üblicher Weise, z. B. durch Neutralisation der Verbindungen der Formel I mit nichttoxischen anorganischen oder organischen Säuren wie z. B.

Salzsäure, Schwefelsäure, Phosphorsäure, Bromwasserstoffsäure, Essigsäure, Milchsäure, Zitronensäure, Äpfelsäure, Salicylsäure, Malonsäure, Maleinsäure oder Bernsteinsäure.

Die erfindungsgemäßen neuen Substanzen der Formel I und ihre Salze können in flüssiger oder fester Form enteral und parenteral appliziert werden. Hierbei kommen alle üblichen Applikationsformen in Frage, beispielsweise Tabletten, Kapseln, Dragees, Sirupe, Lösungen, Suspensionen. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z. B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z. B. Stärke Lactose, Mannit, Methylcellulose, Talkum, hochdisperse Kieselsäuren, höhermolekulare Fettsäuren (wie Stearinsäure), Gelatine Agar-Agar, Calciumphosphat, Magnesiumstearat, tierische und pflanzliche Fette, feste hochmolekulare Polymere (wie Polyethylenglykole) ; für orale Applikation geeignete Zubereitungen können gewünschtenfalls Geschmacks- und Süsstoffe enthalten.

Die Dosierung kann von verschiedenen Faktoren, wie Applikationsweise, Spezies, Alter und/oder individuellen Zustände abhängen. Die täglich zu verabreichenden Dosen liegen bei etwa 0.05-100 mg/kg Körpergewicht.

Bevorzugt im Sinne der vorliegenden Erfindung sind außer den in den Beispielen genannten Verbindungen die folgenden Ester, die nach den beanspruchten Verfahren hergestellt werden können :

Bevorzugte Verbindungen

1. [1-(11-Caproylamido-undecyloxy)-3-propyl]-(2-trimethylammonium-ethyl)-phosphat
2. [1-(11-N-n-Butyl-carbamoyl-undecyloxy)-3-propyl]-(2-trimethylammonium-ethyl)-phosphat
3. [1-(3-Tetradecyloxy-propoxy)-3-propyl]-(2-trimethylammonium-ethyl)-phosphat
4. [2-Cyclopentyl-1-(6-undecyloxy-hexyloxy)-3-propyl]-(2-trimethylammonium-ethyl)-phosphat
5. (1-Octadecyloxy-2-phenyl-3-propyl)-(2-trimethylammonium-ethyl)-phosphat
6. (2-Cyan-1-heptadecyloxy-3-propyl)-(2-trimethylammonium-ethyl)-phosphat
7. (2-Carbamoyl-1-heptadecyloxy-3-propyl)-(2-trimethylammonium-ethyl)-phosphat
8. {2-Hydroxy-1-[5-(3-octyloxy-propoxy)-pentyloxy]-3-propyl}-(2-trimethylammonium-ethyl)-phosphat
9. {2-Methoxy-1-[7-(4-octyl-cyclohexyl)-heptyloxy]-3-propyl}-(2-trimethylammonium-ethyl)-phosphat
10. [2-Isopropoxy-1-(8-nonyloxy-octyloxy)-3-propyl]-(2-trimethylammonium-ethyl)-phosphat
11. [1-Octadecyloxy-2-(2-phenyl-ethoxy)-3-propyl]-(2-trimethylammonium-ethyl)-phosphat
12. [2-(2-Hydroxy-ethoxy)-1-octadecyloxy-3-propyl]-(2-trimethylammonium-ethyl)-phosphat
13. [1-Heptadecyloxy-2-(2-methylmercapto-ethoxy)-3-propyl]-(2-trimethylammonium-ethyl)-phosphat
14. [1-Heptadecyloxy-2-(2-methansulfinyl-ethoxy)-3-propyl]-(2-trimethylammonium-ethyl)-phosphat
15. [1-Heptadecyloxy-2-(2-methansulfonyl-ethoxy)-3-propyl]-(2-trimethylammonium-ethyl)-phosphat
16. [2-(2-Amino-ethoxy)-1-hexadecyloxy-3-propyl]-(2-trimethylammonium-ethyl)-phosphat
17. [2-(2-Acetamido-ethoxy)-1-hexadecyloxy-3-propyl]-(2-trimethylammonium-ethyl)-phosphat
18. [1-Hexadecyloxy-2-(2-methansulfonamido-ethoxy)-2-propyl]-(2-trimethylammonium-ethyl)-phosphat
19. (2-Ethoxycarbonylmethoxy-1-heptadecyloxy-3-propyl)-(2-trimethylammonium-ethyl)-phosphat
20. (2-Cyanmethoxy-1-heptadecyloxy-3-propyl)-(2-trimethylammonium-ethyl)-phosphat
21. (2-N-Dodecyl-carbamoylmethoxy-1-heptadecyloxy-3-propyl)-(2-trimethylammonium-ethyl)-phosphat
22. (1-Hexadecyloxy-2-methylmercapto-3-propyl)-(2-trimethylammonium-ethyl)-phosphat
23. (1-Hexadecyloxy-2-methansulfinyl-3-propyl)-(2-trimethylammonium-ethyl)-phosphat
24. (1-Hexadecyloxy-2-methansulfonyl-3-propyl)-(2-trimethylammonium-ethyl)-phosphat
25. [1-(4-Tridecyloxy-butoxy)-3-cyclopentyl]-(2-trimethylammonium-ethyl)-phosphat
26. S-(1-Hexadecyloxy-2-methoxymethyl-3-propyl)-(2-trimethylammonium-ethyl)-thiophosphat
27. S-(1-Heptadecyloxy-2-isopropoxy-3-propyl)-(2-trimethylammonium-ethyl)-thiophosphat
28. S-(1-Hexadecyloxy-2-methylmercapto-3-propyl)-(2-trimethylammonium-ethyl)-thiophosphat
29. S-(1-Octadecyloxy-3-cyclopentyl)-(2-trimethylammonium-ethyl)-thiophosphat

## Beispiel 1

(2-Methoxymethyl-1-octadecyloxy-3-propyl)-(2-trimethylammonium-ethyl)-phosphat

Zu 1.2 g (3.2 m Mol) 2-Methoxymethyl-3-octadecyloxy-1-propanol in 15 ml absolutem Toluol gibt man bei 0 °C eine Lösung von 1.1 g Triethylamin (11 m Mol) und 1.1 g (4.5 m Mol) 2-Bromethylphosphorsäureester-dichlorid in 15 ml absolutem Toluol. Man läßt 5 h bei 0 °C und über Nacht bei Zimmertemperatur rühren, versetzt die Mischung dann bei 0 °C mit 13 ml 0.1 N Kaliumchloridlösung, rührt 1 h bei 0 °C und 2 h bei Zimmertemperatur kräftig durch, trennt die Phasen, trocknet die organische

mit Natriumsulfat und engt ein. Es bleiben 1.9 g eines gelben Öls zurück, das in 40 ml absolutem Methanol/40 ml absolutem Chloroform gelöst wird. Man leitet bis zur Sättigung trocknes Trimethylamin ein, erhitzt die Lösung 24 h am Rückfluß, engt die Lösung am Rotavapor ein, löst den Rückstand in 70 ml absolutem Methanol, versetzt mit 1 g Silberacetat und läßt 4 h bei Zimmertemperatur rühren. Man saugt ab, wäscht den Rückstand mit absolutem Methanol und engt das Filtrat ein. Es bleibt ein schwarzes Öl, das nach 12 h über 100 g Kieselgel gereinigt wird (Eluationsmittel : Methylenchlorid/Methanol/Wasser im Verhältnis 65/25/4). Die Fraktionen mit dem gewünschten Produkt werden zusammen eingeengt, über Phosphorpentoxyd getrocknet und aus Chloroform/Aceton umgefällt. Man erhält 0.3 g = 17 % einer hygroskopischen Substanz mit einem Fp : 70 °C (Sintern), 248-250 °C unter Zersetzung. Die Verbindung enthält 2 Mol Kristallwasser.

Das als Ausgansmaterial eingesetzte 2-Methoxymethyl-3-octadecyloxy-1-propanol (wachsartig ; Fp. ~ 30 °C) erhält man durch Umsetzung des Mono-natriumsalzes des 2-Methoxymethyl-1.3-propandiols mit Octadecylbromid und anschließender säulenchromatographischer Trennung.

In analoger Weise erhält man durch Verwendung von

a) 2-Ethyl-3-octadecyloxy-1-propanol (wachsartige Substanz) als Ausgangsmaterial den (2-Ethyl-1-octadecyloxy-3-propyl)-(2-trimethylammonium-ethyl)-phosphat × 2 Kristallwasser
in einer Ausbeute von 19 %. Fp : 80 °C (Sintern), 264-265 °C Z.

b) 2-Methoxy-3-(2-methoxy-octadecyloxy)-1-propanol (wachsartige Substanz ; Fp. ≈ 25 °C) als Ausgangsmaterial den [2-Methoxy-1-(2-methoxy-octadecyloxy)-3-propyl]-(2-trimethylammonium-ethyl)-phosphat × 2.5 Kristallwasser
in einer Ausbeute von 22 % ; Fp : 60 °C (Sintern), 238-240 °C Z.

c) 2-Methoxy-3-[7-(4-octyl-phenyl)-heptyloxy]-1-propanol (wachsartige Substanz) als Ausgangsmaterial den {2-Methoxy-1-[7-(4-octyl-phenyl)-heptyloxy]-3-propyl}-(2-trimethylammonium-ethyl)-phosphat × 2 Kristallwasser
in einer Ausbeute von 24 %, Fp : 50 °C (Sintern), 240-243 °C Z.

Das als Ausgangsmaterial eingesetzte 2-Methoxy-3-[7-(4-octylphenyl)-heptyloxy)-1-propanol erhält man auf folgende Weise :

4-Octylbenzol wird mit dem Ethylestersäurechlorid der Pimelinsäure in Gegenwart von Aluminiumchlorid in Nitrobenzol zum 7-(4-Octyl-phenyl)-7-oxo-heptansäureethylester (Fp : 38-40 °C) umgesetzt. Die anschließende Hydrierung mit Palladium auf Kohle in Ethanol unter Zusatz von etwas Perchlorsäure führte zum 7-(4-Octyl-phenyl)-heptansäureethylester (ölige Substanz) die Reduktion mit Lithiumaluminiumhydrid ergab dann das 7-(4-Octyl-phenyl)-heptanol (ölige Substanz).

Umsetzung mit Phosphortribromid lieferte das entsprechende Bromid (Öl), das mit dem Mono-natriumsalz des 2-Methoxy-1.3-propandiols Reaktion gebracht wurde.

d) 2-Methoxy-3-(5-dodecyloxy-pentoxy)-1-propanol (ölige Substanz) als Ausgangsmaterial den [2-Methoxy-1-(5-dodecyl-pentoxy)-3-propyl]-(2-trimethylammonium-ethyl)-phosphat × 1 Kristallwasser
in einer Ausbeute von 31 %, Fp : 75 °C (Sintern), 234-236 °C Z.

e) 2-Methoxy-3-(12-pentoxy-dodecyloxy)-1-propanol (ölige Substanz als Ausgangsmaterial den [2-Methoxy-1-(12-pentoxy-dodecyloxy)-3-propyl]-(2-trimethylammonium-ethyl)-phosphat × 2 Kristallwasser
in einer Ausbeute von 27 %, Fp : 60 °C (Sintern), 220-223 °C Z.

f) 2-Methoxy-3-(5-dodecylmercapto-pentoxy)-1-propanol (wachsartige Substanz) als Ausgangsmaterial den [2-Methoxy-1-(5-dodecylmercapto-pentoxy)-3-propyl]-(2-trimethylammonium-ethyl)-phosphat × 2 Kristallwasser
in einer Ausbeute von 12 %, Fp : 60 °C (Sintern), 227-230 °C Z.

g) 2-Methoxymethyl-2-methyl-3-octadecyloxy-1-propanol (wachsartige Substanz) als Ausgangsmaterial den (2-Methoxymethyl-2-methyl-1-octadecyloxy-3-propyl)-(2-trimethylammonium-ethyl)-phosphat × 4 Kristallwasser
in einer Ausbeute von 33 %. Fp : 100 °C (Sintern), 230-233 °C Z.

Das als Ausgangsmaterial eingesetzte 2-Methoxymethyl-2-methyl-3-octadecyloxy-1-propanol erhält man auf folgende Weise :

2-Methoxymethyl-2-methyl-malonsäurediethylester wird mit Lithiumaluminiumhydrid zum 2-Methoxymethyl-2-methyl-1.3-propandiol ($Kp_{12}$ : 122-124 °C) reduziert und als Mono-natriumsalz mit Octadecylbromid zur Reaktion gebracht.

h) 2.2-Bis-methoxymethyl-3-hexadecyloxy-1-propanol (wachsartige Substanz) als Ausgangsmaterial den (2,2-Bis-methoxymethyl-1-hexadecyloxy-3-propyl)-(2-trimethylammonium-ethyl)-phosphat × 1.5 Mol Kristallwasser
in einer Ausbeute von 31 %. Fp : 60 °C (Sintern), 225-230 °C Z.

Beispiel 2

S-(2-Methoxy-1-octadecyloxy-3-propyl)-(2-trimethylammonium-ethyl)-thiophosphat

Zu 2.87 g 2-Methoxy-3-octadecyloxy-1-propanthiol (wachsartige Substanz) in 50 ml absolutem Toluol tropft man bei 0 °C eine Lösung von 2.76 g Triethylamin und 2.35 g 2-Bromethyl-phosphorsäureesterdichlorid in 30 ml Toluol zu, läßt 5 h bei 0 °C und über Nacht bei Zimmertemperatur rühren, versetzt mit 31 ml 0.1 N Kaliumchloridlösung, rührt kräftig 1 h bei 0 °C und 2 h bei Zimmertemperatur. Die organische Phase wird abgetrennt, getrocknet und eingeengt. Man erhält 2.3 g eines öligen Rückstandes, der in 25 ml absolutem Methanol/25 ml absolutem Chloroform gelöst wird. Die Lösung wird mit trocknem Trimethylamin gesättigt und 20 h unter Rückfluß erhitzt. Man engt ein, löst den Rückstand in absolutem Methanol, versetzt mit 1.2 g Silberacetat, rührt 4 h bei Zimmertemperatur, saugt den Niederschlag ab, wäscht mit absolutem Methanol nach und engt das Filtrat ein. Nach 12 h wird das dunkle Öl über eine Kieselgelsäule gereinigt (Elutionsmittel : Methylenchlorid/Methanol/Wasser im Verhältnis 65/25/4). Die Fraktionen mit dem gewünschten Produkt werden zusammen eingeengt, über Phosphorpentoxyd getrocknet und mit Aceton verrieben. Man erhält 0.55 g = 13 % einer hygroskopischen Substanz mit einem Fp : 65 °C (Sintern), 273-274 °C unter Zersetzung. Die Verbindung enthält 1 Mol Kristallwasser.

Das als Ausgangsmaterial eingesetzte 2-Methoxy-3-octadecyloxy-1-propanthiol erhält man auf folgende Weise :

2-Methoxy-3-octadecyloxy-1-propanol setzt man mit Benzolsulfochlorid in absolutem Pyridin zum 2-Methoxy-3-octadecyloxy-1-propanol-benzolsulfonat (Öl) um, läßt dies mit Thioharnstoff in Butanol zum Isothiuroniumsalz reagieren, das dann mit KOH verseift wird und aus dem mit Salzsäure das Thiol freigesetzt wird.

In analoger Weise erhält man durch Verwendung von

a) 3-Heptadecyloxy-1-propanthiol (Wachs) als Ausgangsmaterial das S-(1-Heptadecyloxy-3-propyl)-(2-trimethylammonium-ethyl)-thiophosphat × 2 Kristallwasser
in einer Ausbeute von 11 %. Fp : 70 °C (Sintern), 254-257 °C Z.

b) 3-(3-Tetradecyloxy-propoxy)-1-propanthiol (Öl) als Ausgangsmaterial das S-[1-(3-Tetradecyloxy-propoxy)-3-propyl]-(2-trimethylammonium-ethyl)-thiophospat 2 × Kristallwasser
in einer Ausbeute von 12 %. Fp : 65 °C (Sintern), 217-220 °C Z.

c) 3-(2-Pentadecylmercapto-ethoxy)-1-propanthiol (Öl) als Ausgangsmaterial das S-[1-(2-Pentadecylmercapto-ethoxy)-3-propyl]-(2-trimethylammonium-ethyl)-thiophosphat × 2,5 Kristallwasser
in einer Ausbeute von 9 %. Fp : 58 °C (Sintern), 153-156 °C Z.

d) 2-(2-Methoxy-ethoxy)-3-octadecyloxy-1-propanthiol (Öl) als Ausgangsmaterial das S-[2-(2-Methoxy-ethoxy)-1-octadecyloxy-3-propyl]-(2-trimethylammonium-ethyl)-thiophosphat × 3 Kristallwasser
in einer Ausbeute von 18 %. Fp : 60 °C (Sintern), 219-221 °C Z.

## Beispiel 3

[1-(12-Pentyloxy-dodecyloxy)-3-propyl]-(2-trimethylammonium-ethyl)-phosphat

Das Mononatriumsalz des 1.12-Dodecandiols wird mit Pentylbromid zum 12-Pentoxy-dodecanol umgesetzt, das, nach Elution an einer Kieselgelsäule mit Ligroin-Methylethylketon 4 : 1, als dünnschichtchromatographisch einheitliches Öl erhalten wird.

Ausbeute : 52 %. Dann bromiert man mit Phosphortribromid in Gegenwart von Pyridin und erhält nach Fraktionierung an einer Kieselgelsäule (Ether-Ligroin 1 : 99 als Elutionsmittel) 47 % DC-einheitliches, öliges 12-Pentoxy-dodecylbromid. Dieses Bromid wird mit dem Mononatriumsalz des 1.3-Propandiols umgesetzt und gibt dann mit 56 % Ausbeute nach Säulenreinigung (Elutionsmittel Ether-Ligro 1 : 1) 3-(12-Pentoxy-dodecyloxy)-propanol vom Fp : 25 °C. Die Phosphorylierung, Hydrolyse und Umsetzung mit Trimethylamin erfolgt in der in Beispiel 1 gegebenen Weise und liefert mit 41 % Ausbeute das gewünschte Produkt vom Fp : 63 °C (Sintern), 240-243 °C Z. Es kristallisiert mit 1 Mol Wasser.

## Beispiel 4

[1-(5-Dodecyloxy-pentyloxy)-3-propyl]-(2-trimethylammonium-ethyl)-phosphat

In analoger Weise wie im Beispiel 3 beschrieben, erhält man die folgenden Zwischenprodukte :
5-Dodecyloxy-pentanol : 46 % Ausbeute, Öl, Elutionsmittel Ether-Ligroin 1 : 1.
5-Dodecyloxy-pentylbromid : 64 % Ausbeute, Öl, Elutionsmittel Ether-Ligroin 1 : 99.
3-(5-Dodecyloxy-pentoxy)-propanol : 55 % Ausbeute, Fp : 25 °C, Elutionsmittel Ether-Ligroin 1 : 1.
Der gewünschte Cholinester wird analog Beispiel 1 hergestellt. Ausbeute : 47 % mit 1 Mol Kristallwasser. Fp : 53 °C (Sintern), 242-245 °C Zers.

## Beispiel 5

[1-(2-Pentadecylmercapto-ethoxy)-3-propyl]-(2-trimethylammonium-ethyl)-phosphat

In analoger Weise wie im Beispiel 3 beschrieben, erhält man die folgenden Zwischenprodukte :
2-Pentadecylmercapto-ethanol, Fp : 44-46 °C, aus dem Natriumsalz des Mercaptoethanols und 9-Brompentadecan. Ausbeute : 97 %. Elutionsmittel Ether-Ligroin 1 : 1.

2-Pentadecylmercapto-ethylbromid, Öl, 93 % Ausbeute. Reinigung wie vorstehend.

3-(2-Pentadecylmercapto-ethoxy)-propanol, Fp : 38-40 °C, Ausbeute 12 %, Reinigung wie vorstehend.

Der gewuenschte Cholinester wird analog Beispiel 1 mit 24 % Ausbeute als Dihydrat vom Zersetzungspunkt 245 °C erhalten.

Beispiel 6

[1-(2-Pentadecyloxy-ethoxy)-3-propyl]-(2-trimethylammonium-ethyl)-phosphat

In analoger Weise wie im Beispiel 3 beschrieben erhält man die folgenden Zwischenprodukte :

2-Pentadecyloxy-ethanol, allmählich erstarrendes Öl, 29 % Ausbeute, Elutionsmittel Ligroin-Ether 1 : 1.

2-Pentadecyloxy-ethylbromid, Öl, 60 % Ausbeute, Elutionsmittel Ether-Ligroin 1 : 99.

3-(2-Pentadecyloxy-ethoxy)-propanol, Fp : 30 °C, Ausbeute 25 %, Elutionsmittel Ether-Ligroin 1 : 2.

Der gewünschte Cholinester wird analog Beispiel 1 erhalten. Ausbeute 21 % nach Säulenreinigung in Form eines klebrigen Produkts. Er liegt als hygroskopisches Monohydrat vor.

Beispiel 7

(1-Hexadecyloxy-carbonyloxy-3-propyl)-(2-trimethylammonium-ethyl)-phosphat

Chlorameisensäure-hexadecylester, aus Cetylalkohol und Phosgen in Gegenwart von Dimethylanilin in 95 % Ausbeute als Öl erhalten wird mit dem Mononatriumsalz des 1.3-Propandiols umgesetzt und an der Kieselgelsäule mit Ligroin-Ether 1 : 1 eluiert. Man erhält so 22 % des Hexadecyl-(3-hydroxypropyl)-kohlensäureesters vom Fp : 28-32 °C, der analog Beispiel 1 phosphoryliert wird. Der gewünschte Cholinester wird mit 32 % Ausbeute erhalten. Das Monohydrat sintert bei 55 °C und schmilzt unter Aufschäumen bei 178 °C.

Beispiel 8

In analoger Weise, wie in Beispiel 1 beschrieben, erhält man durch Verwendung von

a) 2-Allyl-3-octadecyloxy-1-propanol (Öl) als Ausgangsmaterial das (2-Allyl-1-octadecyloxy-3-propyl)-(2-trimethylammonium-ethyl)-phosphat × 2 Kristallwasser
in einer Ausbeute von 41 %. Fp : 70 °C (Sintern), 235-240 °C Z.

b) 3-Hexadecyloxy-2-(2-propinyl)-1-propanol (Öl) als Ausgangsmaterial das [1-Hexadecyloxy-2-(2-propinyl)-3-propyl]-(2-trimethylammonium-ethyl)-phosphat × 1 Kristallwasser
in einer Ausbeute von 22 %. Fp : 60 °C (Sintern), 225-237 °C Z.

Das als Ausgangsmaterial eingesetzte 3-Hexadecyloxy-2-(2-propinyl)-1-propanol wurde durch Umsetzung des Mononatriumsalzes des 2-(2-Propinyl)-1.3-propandiols (Öl) mit Hexadecylbromid erhalten.

c) 2-Benzyloxymethyl-3-heptadecyloxy-1-propanol (Wachs) als Ausgangsmaterial das (2-Benzyloxymethyl-1-heptadecyloxy-3-propyl)-(2-trimethylammonium-ethyl)-phosphat × 2 Kristallwasser
in einer Ausbeute von 38 %. Fp : 60 °C (Sintern), 225-230 °C Z.

Das als Ausgangsmaterial eingesetzte 2-Benzyloxymemthyl-3-heptadecyloxy-1-propanol wurde durch Umsetzung des 2-Isopropyl-5-hydroxymethyl-1.3-dioxan mit Benzylbromid zum 2-Isopropyl-5-benzyloxymethyl-1.3-dioxan (Öl), anschließender Spaltung mit Schwefelsäure und Reaktion mit äquimolarer Menge Heptadecylbromid erhalten.

d) 2-Benzyl-3-octadecyloxy-1-propanol (Fp : 38-41 °C) als Ausgangsmaterial das (2-Benzyl-1-octadecyloxy-3-propyl)-(2-trimethylammonium-ethyl)-phosphat × 3 Kristallwasser
in einer Ausbeute von 20 %. Fp : 65 °C (Sintern), 232-235 °C Z.

e) 3-(10-Heptyloxy-decyloxy)-2-(2-methoxy ethoxy)-1-propanol (Öl) als Ausgangsmaterial das [1-(10-Heptyloxy-decyloxy)-2-(2-methoxy-ethoxy)-3-propyl]-(2-trimethylammonium-ethyl)-phosphat × 2 Kristallwasser
in einer Ausbeute von 20 %. Fp : 70 °C (Sintern), 220-223 °C Z.

f) 3-Heptadecyloxy-2-methylmercaptomethyl-1-propanol (Wachs) als Ausgangsmaterial das (1-Heptadecyloxy-2-methylmercapto-methyl-3-propyl)-(2-trimethylammonium-ethyl)-phosphat × 3 Kristallwasser
in einer Ausbeute von 29 %. Fp : 60 °C (Sintern), 235-240 °C Z.

Das als Ausgangsmaterial eingesetzte 3-Heptadecyloxy-2-methylmercaptomethyl-1-propanol wird auf folgende Weise hergestellt :

2-Isopropyl-4-hydroxymethyl-1.3-dioxan setzt man mit Benzolsulfochlorid zum entsprechenden Benzolsulfonat (Fp : 63-65 °C) um. Durch Reaktion mit Natriummethylmercaptid erhält man das 2-Isopropyl-4-methylmercaptomethyl-1.3-dioxan ($Kp_{0.1}$ : 74-77 °C), aus dem durch Säurebehandlung das 2-Methylmercaptomethyl-1.3-propandiol (Öl) hergestellt wird. Umsetzung mit äquimolaren Mengen Heptadecylbromid führt zum gewünschten Ausgangsmaterial.

Beispiel 9

(2-n-Butylmercapto-1-octadecyloxy-3-propyl)-(2-trimethylammonium-ethyl)-phosphat

Zu 0.6 g (4.2 mmol) 2-Chlor-2-oxo-1,3,2-dioxaphospholan in 15 ml absolutem Methylenchlorid gibt man bei 0 °C 2 ml Triethylamin in 15 ml absolutem Methylenchlorid und 1.1 g (2.65 mmol) 2-n-Butylmercapto-3-octadecyloxy-1-propanol (Wachs) in 15 ml absolutem Methylchlorid gelöst. Man läßt 1 Stunde bei 0 °C und dann über Nacht bei Zimmertemperatur rühren, versetzt mit 10 ml Wasser, trennt die organische Phase, trocknet und engt ein. Den Rückstand löst man in 30 ml absolutem Acetonitril, leitet 10 min trocknes Trimethylamin ein und erhitzt die Lösung 12 Stunden im Autoklaven auf 70 °C. Nach dem Abkühlen saugt man den Niederschlag ab, spült mit Acetonitril nach, löst den Niederschlag in Methanol und engt ein. Den Rückstand reinigt man über 100 g Kieselgel (Elutionsmittel : Methylenchlorid/Methanol/Wasser i. V. 65/25/4). Die Fraktionen mit dem gewünschten Produkt werden zusammen eingeengt, über Phosphorpentoxid getrocknet und mit Aceton verrieben. Man erhält 0.33 g ≙ 21 % der gewünschten Substanz mit einem Fp : 60 °C (Sintern), 217-220 °C. Die Verbindung enthält 1 mol Kristallwasser.

Das als Ausgangsmaterial eingesetzte 2-n-Butylmercapto-3-octadecyloxy-1-propanol erhält man auf folgender Weise :

2-Benzyloxy-3-octadecyloxy-1-propanol setzt man mit Dihydropyran zum entsprechenden Tetrahydropyranylderivat (Öl) um, hydriert die Benzylschutzgruppe mit Palladium/Kohle als Katalysator ab, stellt hieraus durch Umsetzung mit Benzolsulsfochlorid das 2-Benzolsulfonyloxy-3-octadecyloxy-1-tetrahydropyranyloxy-propan (Wachs) her. Umsetzung mit dem Natriumsalz des n-Butylmercaptans ergibt das 2-n-Butylmercapto-3-octadecyloxy-1-tetrahydropyranyloxy-propan (Öl), von dem die Tetrahydropyranylschutzgruppe unter sauren Bedingungen abgespalten wird.

In analoger Weise erhält man durch Verwendung von 2-Hexadecylmercapto-3-methoxy-1-propanol (Wachs) als Ausgangsmaterial das

a) (2-Hexadecylmercapto-1-methoxy-3-propyl)-(2-trimethylammonium-ether)-phosphat × 2.5 Kristallwasser
in einer Ausbeute von 22 %. Fp : 179 °C (Sintern), 195-215 °C.

Beispiel 10

[2-Hydroxy-1-(3-tetradecyloxy-propoxy)-3-propyl]-(2-trimethylammonium-ethyl)-phosphat

0.9 g 2-Benzyloxy-3-(3-tetradecyloxy-propoxy)-1-propanolphosphorsäure-mono-cholinester (Fp : 65 °C (Sintern), 238-240 °C) werden in 200 ml Ethanol mit 0.9 g Palladium/Kohle als Katalysator bei Zimmertemperatur hydriert. Nach Beendigung der Wasserstoffaufnahme wird abgesaugt, das Filtrat eingeengt, über Phosphorpentoxid getrocknet und der Rückstand mit Aceton verrieben. Man erhält 0.45 ≙ 60 % der gewünschten Verbindung mit einem Fp : 70 °C (Sintern), 260-262 °C Z. Die Substanz enthält 1 Mol Kristallwasser.

Der als Ausgangsmaterial eingesetzte 2-Benzyloxy-3-(3-tetradecyloxy-propoxy)-1-propanol-phosphorsäure-monocholinester wurde folgendermaßen hergestellt :

Zu 1 ml Phosphoroxytrichlorid in 8 ml absolutem Tetrahydrofuran tropft man bei 0 °C eine Lösung von 3.5 g 2-Benzyloxy-3-(3-tetradecyloxy-propoxy)-1-propanol (Öl) in 40 ml absolutem Tetrahydrofuran. Man läßt 1 h bei O °C und 1 h bei Zimmertemperatur rühren, kühlt ab, versetzt mit 16 ml 1 N Natronlauge und läßt weitere 2 Stunden bei Zimmertemperatur rühren. Man gibt 40 ml alkoholische Salzsäure, 80 ml Chloroform und 40 ml Wasser zu, trennt die organische Phase ab und engt ein. Den Rückstand erhitzt man 4 Stunden in 80 ml 80proz. wässrigen Dioxan unter Rückfluß, engt ein, nimmt den Rückstand in 40 ml Chloroform auf und schüttelt die Lösung mit 20 ml 2 N Salzsäure aus. Die organische Phase wird abgetrennt, getrocknet und eingeengt. Man erhält 3.4 g ≙ 77 % des Phosphorsäure-mono-[2-benzyloxy-3-(3-tetradecycloxy-propoxy-1-propyl]-esters als wachsartige Substanz.

Diese 3.4 g löst man in 100 ml absolutem Pyridin, gibt 0.8 g 2-Bromethanol und 4.5 g 2,4,6-Triisopropylbenzol-sulfochlorid zu. Man läßt über Nacht bei Zimmertemperatur rühren, kühlt ab, versetzt mit 40 ml 0.1 N wässriger Kaliumchloridlösung, läßt 2 Stunden rühren, säuert mit konzentrierter Salzsäure an, schüttelt mit Methylenchlorid aus, trocknet die organische Phase und engt ein. Man erhält 3.9 g ≙ 95 % des Phosphorsäure-(2-bromethyl)-[2-benzyloxy-3-(3-tetradecyloxy-propoxy)-1-propyl]-esters als ölige Substanz.

Die 3.9 g werden in 50 ml Methanol/50 ml Chloroform gelöst. Man leitet 10 min trocknes Trimethylamin ein, erhitzt 20 h unter Rückfluß, engt ein, nimmt den Rückstand in 75 ml Methanol auf, versetzt mit 1.5 g Silberacetat, läßt 2 Stunden rühren, saugt ab und engt ein. Den Rückstand reinigt man über 100 g Kieselgel (Elutionsmittel, Methylenchlorid/Methanol/Wasser i. V. 65/25/4).

Man erhält 1.4 g ≙ 35 % der gewünschten Substanz mit einem Fp : 65 °C (Sintern), 238-240 °C. Die Verbindung enthält 2 mol Kristallwasser.

In analoger Weise erhält man durch Verwendung von 2-Benzyloxymethyl-3-heptadecyloxy-1-propanol-phosphorsäure-mono-cholinester (Fp : 60 °C (Sintern), 225-230 °C Z.)) (s. Beispiel 10 c) als Ausgangsmaterial das

a) (1-Heptadecyloxy-2-hydroxy-3-propyl)-(2-trimethylammonium-ethyl)-phosphat × 4 Kristallwasser in einer Ausbeute von 57 %. Fp : 60 °C (Sintern), 228-230 °C Z.

Versuchsprotokoll

a) Prüfung auf Tumorzell-Zytotoxizität

Die neuen Substanzen wurden hinsichtlich ihrer zytotoxischen Wirkung auf Tumorzellen der Maus in einem Screening untersucht. Als Target-Zellen wurden einmal Zellen eines durch Methylcholanthreninduzierten Tumors (MethA), der als Ascites in der Maus passagiert wird, zum anderen Abelsen-8.1-Lymphomzellen (Abls), die in vitro in Kultur gehalten werden, benutzt.

Zum Screenen wurden 5 × $10^4$/ml dieser Zellen in Dulbecco's Modified Eagle's Medium, das mit 10 % hitzeinaktiviertem fetalem Kälberserum, 5 × $10^{-5}$ m Mercaptoäthanol, 50 U Penicillin und 50 µg Streptomycin/ml angereichert ist, und verschiedenen Konzentrationen der neuen schwefelhaltigen Phospholipide zusammen für 24 Std. kultiviert. Die Kultivation fand bei 95 % Luftfeuchtigkeit, 37 °C, 10 % $Co_2$ im Brutschrank statt.

Die Wirkung der Substanzen wurde durch den Vergleich des Wachstums der Tumorzellen im Medium mit den neuen schwefelhaltigen Phospholipiden zu einer Kontrollkultur ohne Substanz bestimmt. Als Vergleichssubstanz wurde bei jedem Versuch 1-Octadecyl-2-methylglycero-3-phosphorylcholin mitgeführt (Verbindung A).

Das Wachstum der MethA-Zellen wurde durch den Einbau von $^3$H-Thymidin in die DNA der Zellen gemessen ; das der Abls-Zellen durch die Bestimmung der alkalischen Phosphatase-Aktivität eines Aliquot der Kulturen. Für jede Substanz wurde die Konzentration berechnet, bei der der Thymidineinbau bzw. die alkalische Phosphatase-Aktivität gegenüber einer unbehandelten Tumorzellkontrolle um 50 % reduziert ist.

In der nachstehenden Tabelle sind die Ergebnisse der getesteten Substanzen aufgeführt. Die relative Wirksamkeit dieser Substanzen ist durch einen Faktor ausgedrückt, der das Verhältnis der Vergleichssubstanz zu den untersuchten neuen Phospholipiden darstellt. Die Substanz A stellt die Vergleichssubstanz dar.

b) Prüfung auf Thrombozytenaggregation

Hinsichtlich der plättchenaggregierenden Wirkung der neuen Substanzen wurde Blut von gesunden Menschen bei 100 g 10 Minuten abzentrifugiert, um das plättchenreiche Plasma für die Aggregationsprüfung zu gewinnen. In einem Aggregometer wurde das Plasma auf 37 °C erwärmt und dann die Substanzen hinzugegeben. Die in der Tabelle aufgeführten Werte repräsentieren die Konzentration der Substanzen, die eine irreversible Plättchenaggregation auslösen.

Tabelle

| | Tumorzell-zytotoxizität Faktor | Plättchen-aggregation mg/ml |
|---|---|---|
| Verbind. A | 1.0 | $<$ 0.05 |
| Bsp. 1 a) | 1.0 | $>$ 2 |
| Bsp. 1 g) | 0.8 | $>$ 2 |
| Bsp. 1 h) | 0.9 | $>$ 1 |
| Bsp. 2 | 1.0 | $>$ 1 |
| Bsp. 6 | 0.60 | $>$ 1 |

$$\text{Faktor} = \frac{C_{50}\ \text{Verbindung A}}{C_{50}\ \text{Verbindung X}}$$

**Patentansprüche** (für die Vertragsstaaten : BE, CH, DE, FR, GB, IT, LI, LU, NL, SE)

1. Phospholipide der allgemeinen Formel I

$$R_1-X-R_2-Y-R_3-Z-\underset{O^{\ominus}}{\overset{O}{\overset{\|}{P}}}-O-R_4-\overset{\oplus}{N}(R_5)_3 \qquad (I)$$

in der

X = Valenzstrich, Sauerstoff, Schwefel, die Sulfonyl-, die Sulfinylgruppe, ein Phenylen-, ein $C_3$-$C_8$ Cycloalkylenrest, die —CONH—, —NHCO—, —NHCONH— oder Carbonylgruppe,

$R_1$ = Wasserstoff oder ein geradkettiger oder verzweigter, gesättigter oder ungesättigter Alkylrest mit 1-18 bzw. 2-18 Kohlenstoffatomen, der gegebenenfalls ein- oder mehrfach durch Halogen, eine Alkoxy-, Alkylmercapto-, Alkansulfinyl-, Alkansulfonyl-, Carbalkoxygruppe oder den Phenylrest substituiert ist,

$R_2$ = eine geradkettige oder verzweigte gesättigte oder ungesättigte Alkylenkette mit 1-18 bzw. 2-18 Kohlenstoffatomen, die gegebenenfalls ein- oder mehrfach durch Halogen, eine Alkoxy-, Alkylmercapto-, Alkansulfinyl-, Alkansulfonyl-, Carbalkoxygruppe oder den Phenylrest substituiert ist,

Y = Sauerstoff, O—CO—O, O—CO—NH, O—CS—NH,

$R_3$ = $—CH_2—CH_2—CH_2—$ die auch Teil eines Cyclopentan-, Cyclohexan- oder Cycloheptan-Ringsystems sein kann und die gegebenenfalls ein- oder mehrfach durch Hydroxy, Halogen, eine Alkylmercapto-, Alkansulfinyl-, Alkansulfonylgruppe, die Nitril-, Alkoxycarbonyl- oder eine gegebenenfalls durch Alkylgruppen substituierte Carbamoylgruppe, einen Cyclopentyl-, Cyclohexyl- oder Cycloheptyl-Rest-, einen gegebenenfalls substituierten Phenylrest oder eine Alkoxygruppe substituiert ist, die ihrerseits gegebenenfalls durch Phenyl, Hydroxy, eine Alkoxy-, Alkylmercapto-, Alkansulfinyl-. Alkansulfonyl-, eine gegebenenfalls acylierte Aminogruppe, einen Alkoxycarbonyl-, die Nitril- oder einen gegebenenfalls durch Alkylgruppen substituierten carbamoylrest substituiert ist,

Z = Sauerstoff oder Schwefel,

$R_4$ = eine geradkettige oder verzweigte Alkylenkette mit 2-5 Kohlenstoffatomen,

$R_5$ = Wasserstoff oder eine niedere Alkylgruppe bedeuten,

wobei für den Fall, daß die Gruppe $R_1$-X-$R_2$ eine unsubstituierte geradkettige oder verzweigte, gesättigte oder ungesättigte Alkylkette mit 1-18 bzw. 2-18 Kohlenstoffatomen und Z Sauerstoff darstellten,

$R_3$ = nicht gleich eine Propylenkette sein darf, die gegebenenfalls durch Hydroxy, Alkoxy oder Benzyloxy substituiert ist,

und wobei für den Fall, daß die Gruppe $R_1$-X-$R_2$ eine durch Halogen oder Phenyl substituierte Alkylkette mit 1-18 Kohlenstoffatomen darstellt und, Y und Z = Sauerstoff bedeuten,

$R_3$ = nicht gleich eine Propylen- oder 2-Hydroxypropylenkette sein darf,

sowei deren pharmakologisch unbedenkliche Salze.

2. Verbindungen der Formel Ia

$$C_{18}H_{37}-O-R_{3a}-O-\underset{O^{\ominus}}{\overset{O}{\overset{\|}{P}}}-O-CH_2CH_2\overset{\oplus}{N}(CH_3)_3 \qquad (Ia)$$

in der

$$R_{3a} = -CH_2-\underset{CH_2-OCH_3}{\underset{|}{CH}}-CH_2- \;;\; -CH_2-\underset{C_2H_5}{\underset{|}{CH}}-CH_2- \;;\; -CH_2-\underset{CH_2-C_6H_5}{\underset{|}{CH}}-CH_2- \;;$$

$$-CH_2-\overset{CH_3}{\underset{CH_2OCH_3}{\underset{|}{\overset{|}{C}}}}-CH_2- \;;\; -CH_2-\underset{CH_2-CH=CH_2}{\underset{|}{CH}}-CH_2-$$

bedeutet,

sowie deren pharmakologisch unbedenkliche Salze.

3. Verbindungen der Formel Ib

$$C_{17}H_{35}-O-R_{3b}-O-\underset{O^{\ominus}}{\overset{O}{\overset{\|}{P}}}-O-CH_2-CH_2\overset{\oplus}{N}(CH_3)_3 \qquad (Ib)$$

in der

$$R_{3b} = -CH_2-\underset{CH_2-O-CH_2-C_6H_5}{\underset{|}{CH}}-CH_2 \;;\; -CH_2-\underset{CH_2-SCH_3}{\underset{|}{CH}}-CH_2 \;;$$

$$-CH_2-\underset{CH_2OH}{\underset{|}{CH}}-CH_2- \;;\; -CH_2-\underset{CH_2-SO_2-CH_3}{\underset{|}{CH}}-CH_2-$$

bedeutet,
sowie deren pharmakologisch unbedenkliche Salze.

4. Verbindungen der Formel Ic

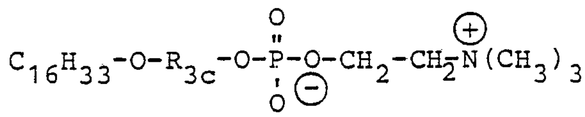 (Ic)

in der

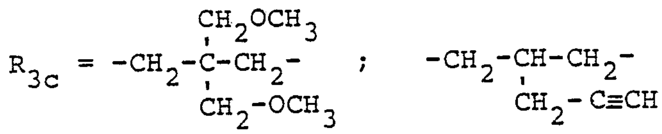

bedeutet,
sowie deren pharmakologisch unbedenklichen Salze.

5. [2-Methyl-1-(3-tetradecyloxy-propoxy)-3-propyl]-(2-trimethylammonium-ethyl)-phosphat sowie deren pharmakologisch unbedenkliche Salze.

6. Verfahren zur Herstellung von Verbindungen gemäß Anspruch 1, dadurch gekennzeichnet, daß man in an sich bekannter Weise eine Verbindung der allgemeinen Formel II

$$R_1—X—R_2—Y—R_3—ZH \quad (II)$$

entweder

a) mit einer Verbindung der allgemeinen Formel III oder IIIa

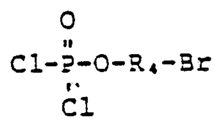 (III) 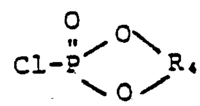 (IIIa),

in Gegenwart eines säurebindenden Mittels umsetzt, das Reaktionsprodukt bei Verwendung einer Verbindung der Formel III selektiv hydrolysiert und das verbleibende Bromatom gegen eine gegebenenfalls alkylierte Ammoniumgruppe austauscht, bei Verwendung einer Verbindung der Formel IIIa direkt mit gegebenenfalls alkyliertem Ammoniak behandelt
oder

b) in eine Verbindung der allgemeinen Formel IV überführt

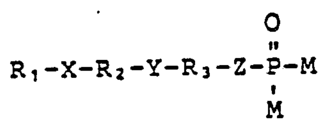 (IV),

in der M Hydroxy, Chlor, Brom oder Alkylmercapto bedeuten, und diese mit einer Verbindung der allgemeinen Formel V

$$HO—R_4—T \quad (V),$$

in der T Chlor oder Brom oder die Gruppe $N(R_5)_3{}^+Hal^-$ darstellt, wobei $R_5$ die oben angegebene Bedeutung hat und $Hal^-$ Chlorid, Bromid oder Jodid sein soll, umsetzt und für den Fall, daß T Chlor oder Brom ist, das so erhaltene Zwischenprodukt anschließend mit einem Amin $N(R_5)_3$ quaternisiert,
oder

c) mit einer Verbindung der allgemeinen Formel VI

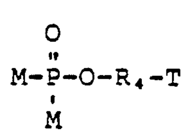 (VI),

in Gegenwart eines säure- oder wasserbindenden Mittels umsetzt,
die erhaltenen Verbindungen gewünschtenfalls in das innere Salz überführt, gegebenenfalls zu den Sulfonen oder Sulfoxiden oxidiert, gegebenenfalls quaternisiert und gewünschtenfalls Verbindungen der Formel I in pharmakologisch unbedenkliche Salze überführt.

7. Arzneimittel, enthaltend eine der Verbindungen der Ansprüche 1 bis 5 und übliche Träger- und Hilfsstoffe.

8. Verbindungen gemäß Ansprüchen 1 bis 5 zur Verwendung bei der Bekämpfung von Krebs.

**Patentansprüche** (für den Vertragsstaat AT)

1. Verfahren zur Herstellung von Verbindungen der Formel I

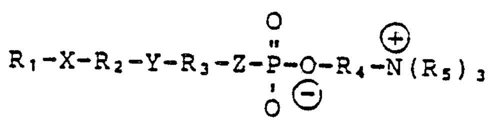

(I),

in der

X = Valenzstrich, Sauerstoff, Schwefel, die Sulfonyl-, die Sulfinylgruppe, ein Phenylen-, ein $C_3$-$C_8$ Cycloalkylenrest die —CONH—, —NHCO—, —NHCONH— oder Carbonylgruppe,

$R_1$ = Wasserstoff oder ein geradkettiger oder verzweigter, gesättigter oder ungesättigter Alkylrest mit 1-18 bzw. 2-18 Kohlenstoffatomen, der gegebenenfalls ein- oder mehrfach durch Halogen, eine Alkoxy-, Alkylmercapto-, Alkansulfinyl-, Alkansulfonyl-, Carbalkoxygruppe oder den Phenylrest substituiert ist,

$R_2$ = eine geradkettige oder verzweigte gesättigte oder ungesättigte Alkylenkette mit 1-18 bzw. 2-18 Kohlenstoffatomen, die gegebenenfalls ein- oder mehrfach durch Halogen, eine Alkoxy, Alkylmercapto-, Alkansulfinyl-, Alkansulfonyl-, Carbalkoxygruppe oder den Phenylrest substituiert ist,

Y = Sauerstoff, O—CO—O, O—CO—NH, O—CS—NH,

$R_3$ = —$CH_2$—$CH_2$—$CH_2$—

die auch Teil eines Cyclopentan-, Cyclohexan- oder Cycloheptan-Ringsystems sein kann und die gegebenenfalls ein- oder mehrfach durch Hydroxy, Halogen, eine Alkylmercapto-, Alkansulfinyl-, Alkansulfonylgruppe, die Nitril-, Alkoxycarbonyl- oder eine gegebenenfalls durch Alkylgruppen substituierte Carbamoylgruppe, einen Cyclopentyl-, Cyclohexyl- oder Cycloheptyl- Rest-, einen gegebenenfalls substituierten Phenylrest oder eine Alkoxygruppe substituiert ist, die ihrerseits gegebenenfalls durch Phenyl, Hydroxy, eine Alkoxy-, Alkylmercapto-, Alkansulfinyl-, Alkansulfonyl-, eine gegebenenfalls acylierte Aminogruppe, einen Alkoxycarbonyl-, die Nitril- oder einen gegebenfalls durch Alkylgruppen substituierten Carbamoylrest substituiert ist,

Z = Sauerstoff oder Schwefel,

$R_4$ = eine geradkettige oder verzweigte Alkylenkette mit 2-5 Kohlenstoffatomen,

$R_5$ = Wasserstoff oder eine niedere Alkylgruppe bedeuten,

wobei für den Fall, daß die Gruppe $R_1$—X—$R_2$ zusammen eine unsubstituierte geradkettige oder verzweigte, gesättigte oder ungesättigte Alkylkette mit 1-18 bzw. 2-18 Kohlenstoffatomen und Z Sauerstoff darstellten,

$R_3$ = nicht gleich eine Propylenkette sein darf, die gegebenenfalls durch Hydroxy, Alkoxy oder Benzyloxy substituiert ist,

und wobei für den Fall, daß die Gruppe $R_1$—X—$R_2$ eine durch Halogen oder Phenyl substituierte Alkylkette mit 1-18 Kohlenstoffatomen darstellt und Y und Z = Sauerstoff bedeuten,

$R_3$ = nicht gleich eine Propylen- oder 2-Hydroxy-propylenkette sein darf,

sowie deren pharmakologisch unbedenklichen Salze, dadurch gekennzeichnet, daß man eine Verbindung der allgemeinen Formel II

$$R_1—X—R_2—Y—R_3—ZH \quad (II)$$

entweder

a) mit einer Verbindung der allgemeinen Formel III oder IIIa

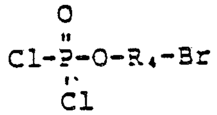

(III)

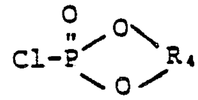

(IIIa),

in Gegenwart eines Säurebindenden Mittels umsetzt, das Reaktionsprodukt bei Verwendung einer Verbindung der Formel III selektiv hydrolysiert und das verbleibende Bromatom gegen eine gegebenenfalls alkylierte Ammoniumgruppe austauscht, bei Verwendung einer Verbindung der Formel IIIa direkt mit gegebenenfalls alkyliertem Ammoniak behandelt

oder

b) in eine Verbindung der allgemeinen Formel IV überführt

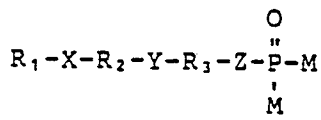

(IV),

in der M Hydroxy, Chlor, Brom oder Alkylmercapto bedeuten, und diese mit einer Verbindung der allgemeinen Formel V

$$HO—R_4—T \qquad (V),$$

in der T Chlor oder Brom oder die Gruppe $N(R_5)_3^+Hal^-$ darstellt, wobei $R_5$ die oben angegebene Bedeutung hat und $Hal^-$ Chlorid, Bromid oder Jodid sein soll, umsetzt und für den Fall, daß T Chlor oder Brom ist, das so erhaltene Zwischenprodukt anschließend mit einem Amin $N(R_5)_3$ quaternisiert, oder

c) mit einer Verbindung der allgemeinen Formel VI

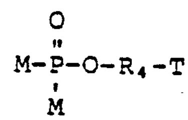

(VI),

in Gegenwart eines säure- oder wasserbindenden Mittels umsetzt, die erhaltenen Verbindungen gewünschtenfalls in das innere Salz überführt, gegebenenfalls zu den Sulfonen oder Sulfoxiden oxidiert, gegebenenfalls quaternisiert und gewünschtenfalls Verbindungen der Formel I in pharmakologisch unbedenkliche Salze überführt.

2. Verfahren zur Herstellung von Verbindungen der Formel Ia

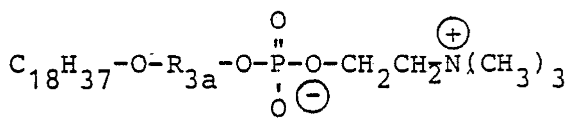

(Ia),

in der

$R_{3a}$ =
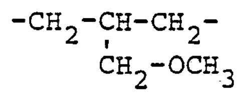
;
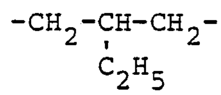
;
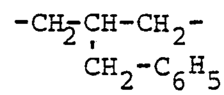
;
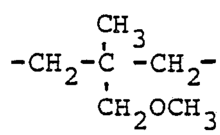
;
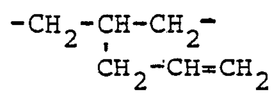

bedeutet,

sowie deren pharmakologisch unbedenklichen Salze gemäß Anspruch 1, wobei die Gruppe $R_1—X—R_2 = C_{18}H_{37}$, $R_3 = R_{3a}$, Y = Sauerstoff, Z = Sauerstoff, $R_4 = —CH_2CH_2—$ und $R_5 = —CH_3$ bedeuten.

3. Verfahren zur Herstellung von Verbindungen der Formel Ib

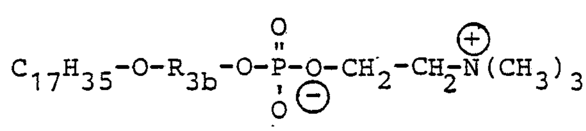

(Ib),

in der

$R_{3b}$ =
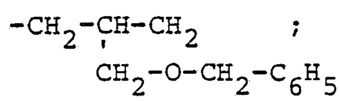
;
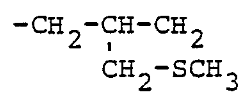
;
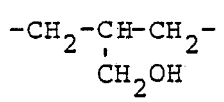
;
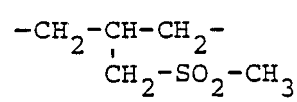

bedeutet,

sowie deren pharmakologisch unbedenklichen Salze gemäß Anspruch 1, wobei die Gruppe

$R_1—X—R_2 = C_{17}H_{35}$, $R_3 = R_{3b}$, Y = Sauerstoff, X = Sauerstoff, $R_4 = —CH_2CH_2—$ und $R_5 = —CH_3$ bedeuten.

4. Verfahren zur Herstellung von Verbindungen der Formel Ic

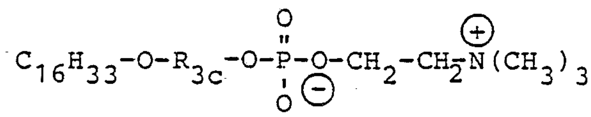
(Ic),

in der

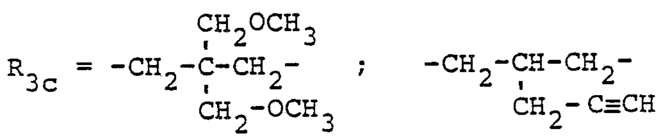

bedeutet,
sowie deren pharmakologisch unbedenklichen Salze gemäß Anspruch 1, wobei die Gruppe $R_1—X—R_2 = C_{16}H_{33}$, $R_3 = R_{3c}$, Y = Sauerstoff, X = Sauerstoff, $R_4 = —CH_2CH_2—$ und $R_5 = —CH_3$ bedeuten.

5. Verfahren zur Kerstellung von [2-Methyl-1-(3-tetradecyloxy-propoxy)-3-propyl]-(2-trimethylammonium-ethyl)-phosphat sowie deren pharmakologisch unbedenklichen Salze gemäß Anspruch 1, wobei die Gruppe $R_1—X—R_2 = C_{14}H_{29}—O—(CH_2)_3—$, $R_3$ die Gruppe $—CH_2—CH_2—CH_2—$, Y = Sauerstoff, Z = Sauerstoff, $R_4 = —CH_2CH_2—$ und $R_5 = —CH_3$ bedeuten.

**Claims** (for the Contracting States : BE, CH, DE, FR, GB, IT, LI, LU, NL, SE)

1. Phospholipids of the general formula I

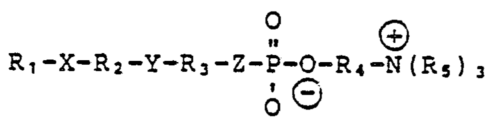
(I)

in which

X signifies a valency bond, oxygen, sulphur, the sulphonyl, the sulphinyl, a phenylene, a $C_3$-$C_8$ cycloalkylene radical, the —CONH—, —NHCO—, —NHCONH— or carbonyl group,

$R_1$ hydrogen or a straight-chained or branched, saturated or unsaturated alkyl radical with 1-18 or 2-18 carbon atoms, which is possibly substituted one or more times by halogen, an alkoxy, alkylmercapto, alkanesulphinyl, alkanesulphonyl, carbalkoxy group or the phenyl radical,

$R_2$ a straightchained or branched, saturated or unsaturated alkylene chain with 1-18 or 2-18 carbon atoms, which is possibly substituted one or more times by halogen, an alkoxy, alkylmercapto, alkanesulphinyl, alkanesulphonyl, carbalkoxy group or the phenyl radical,

Y oxygen, O—CO—O, O—CO—NH, O—CS—NH group,

$R_3$ $—CH_2CH_2CH_2$ which can also be part of a cyclopentane, cyclohexane or cycloheptane ring system and which is possibly substituted one or more times by hydroxyl, halogen, an alkylmercapto, alkanesulphinyl, alkanesulphonyl, the nitrile, alkoxycarbonyl or a carbamoyl group possibly substituted by alkyl groups, a cyclopentyl, cyclohexyl or cycloheptyl radical, a possibly substituted phenyl radical or an alkoxy group which in turn is possibly substituted by phenyl, hydroxyl, an alkoxy, alkylmercapto, alkanesulphinyl, alkanesulphonyl, a possibly acylated amino group, an alkoxycarbonyl, the nitrile or a carbamoyl radical possibly substituted by alkyl groups,

Z oxygen or sulphur,

$R_4$ a straight-chained or branched alkylene chain with 2-5 carbon atoms,

$R_5$ hydrogren or a lower alkyl group,

whereby for the case that the group $R_1—X—R_2$ represents an unsubstituted straight -chained or branched, saturated or unsaturated alkyl chain with 1-18 or 2-18 carbon atoms and Z represents oxygen,

$R_3$ cannot represent a propylene chain which is possibly substituted by hydroxyl, alkoxy or benzyloxy, and whereby for the case that the group $R_1—X—R_2$ represents an alkyl chain with 1-18 carbon atoms substituted by halogen or phenyl and Y and Z = oxygen,

$R_3$ cannot represent a propylene or 2-hydroxypropylene chain ;

as well as their pharmacologically acceptable salts.

2. Compounds of the formula Ia

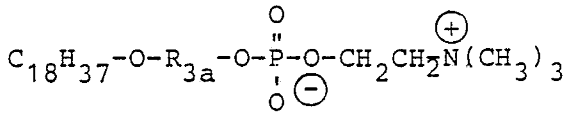

(Ia)

in which $R_{3a}$ signifies

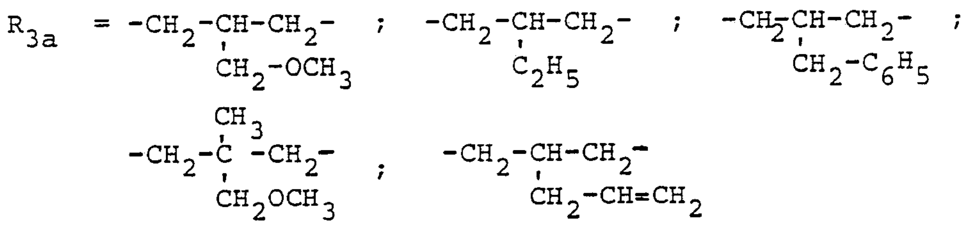

as well as their pharmacologically acceptable salts.

3. Compounds of the formula Ib

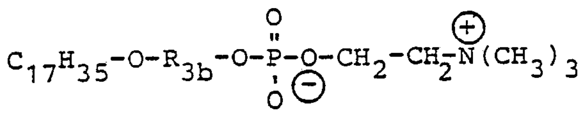

(Ib)

in which $R_{3b}$ signifies

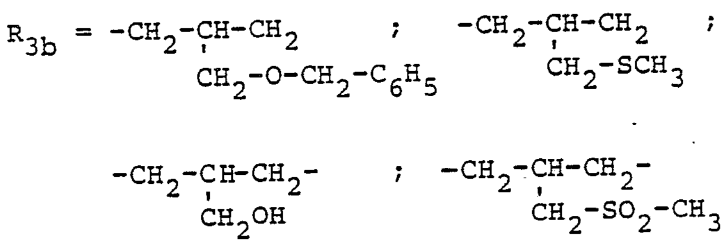

as well as their pharmacologically acceptable salts.

4. Compounds of the formula Ic

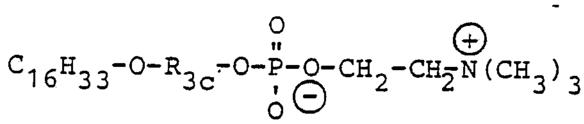

(Ic)

in which $R_{3c}$ signifies

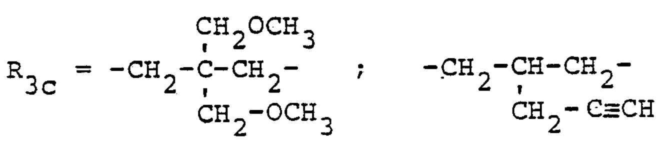

as well as their pharmacologically acceptable salts.

5. [2-Methyl-1-(3-tetradecyloxy-propoxy)-3-propyl]-(2-trimethylammonium-ethyl) phosphate, as well as their pharmacologically acceptable salts.

6. Process for the preparation of compounds according to claim 1, characterised in that in per se known manner, a compound of the general formula II

$$R_1—X—R_2—Y—R_3—ZH \quad (II)$$

is either

a) reacted with a compound of the general formula III or IIIa

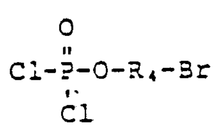

(III)

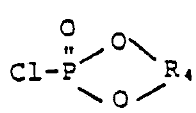

(IIIa)

in the presence of an acid-binding agent, the reaction product, when using a compound of the formula III, is selectively hydrolysed and the remaining bromine atom exchanged for a possibly alkylated ammonium group, when using a compound of the formula IIIa is treated directly with possibly alkylated ammonia ; or

b) converted into a compound of the general formula IV

$$R_1-X-R_2-Y-R_3-Z-\overset{\overset{\displaystyle O}{\|}}{\underset{\underset{\displaystyle M}{|}}{P}}-M \qquad (IV)$$

in which M signifies hydroxyl, chlorine, bromine or alkylmercapto, and this reacted with a compound of the general formula V

$$HO—R_4—T \qquad (V)$$

in which T represents chlorine or bromine or the group $N(R_5)_3{}^+Hal^-$, whereby $R_5$ has the above-given meaning and $Hal^-$ is to be chloride, bromide or iodide, and when T is chlorine or bromine, the so obtained intermediate is subsequently quaternised with an amine $N(R_5)_3$, or

c) reacted with a compound of the general formula VI

$$M-\overset{\overset{\displaystyle O}{\|}}{\underset{\underset{\displaystyle M}{|}}{P}}-O-R_4-T \qquad (VI)$$

in the presence of an acid- or water-binding agent ; the compounds obtained converted, if desired, into the inner salt, possibly oxidised to the sulphones or sulphoxides, possibly quaternised and, if desired, compounds of the formula I converted into pharmacologically acceptable salts.

7. Medicaments, containing one of the compounds of claims 1 to 5 and conventional carrier and adjuvant materials.

8. Compounds according to claims 1 to 5 for use in the combating of cancer.

**Claims** ( for the Contracting State AT)

1. Process for the preparation of compounds of the formula I

$$R_1-X-R_2-Y-R_3-Z-\overset{\overset{\displaystyle O}{\|}}{\underset{\underset{\displaystyle O^{\ominus}}{|}}{P}}-O-R_4-\overset{\oplus}{N}(R_5)_3 \qquad (I),$$

in which

X = valency bond, oxygen, sulphur, the sulphonyl, the sulphinyl, a phenylene, a $C_3$-$C_8$ cycloalkylene radical, the —CONH—, —NHCO—, —NHCONH— or carbonyl group,

$R_1$ = hydrogen or a straight-chained or branched, saturated or unsaturated alkyl radical with 1-18 or 2-18 carbon atoms which is possibly substituted one or more times by halogen, an alkoxy, alkylmercapto, alkanesulphinyl, alkanesulphonyl, carbalkoxy group or the phenyl radical,

$R_2$ = a straight-chained or branched, saturated or unsaturated alkylene chain with 1-18 or 2-18 carbon atoms, which is possibly substituted one or more times by halogen, an alkoxy, alkylmercapto, alkanesulphinyl, alkanesulphonyl, carbalkoxy group or the phenyl radical,

Y = oxygen, O—CO—O, O—CO—NH, O—CS—NH,

$R_3$ = —$CH_2$—$CH_2$—$CH_2$—

which can also be part of a cyclopentane, cyclohexane or cycloheptane ring system and which is possibly substituted one or more times by hydroxyl, halogen, an alkylmercapto, alkanesulphinyl, alkanesulphonyl group, the nitrile, alkoxycarbonyl or a carbamoyl group possibly substituted by alkyl groups, a cyclopentyl, cyclohexyl or cycloheptyl radical, a possibly substituted phenyl radical or an alkoxy group which, in turn, is possibly substituted by phenyl, hydroxyl, an alkoxy, alkylmercapto, alkanesulphinyl, alkanesulphonyl, a possibly acylated amino group, an alkoxycarbonyl, the nitrile or a carbamoyl radical possibly substituted by alkyl groups,

Z = oxygen or sulphur,

$R_4$ = a straight-chained or branched alkylene chain with 2-5 carbon atoms,

$R_5$ = hydrogen or a lower alkyl group,

whereby for the case in which the group $R_1$—X—$R_2$ represents an unsubstituted, straight-chained or branched, saturated or unsaturated alkyl chain with 1-18 or 2-18 carbon atoms and Z represents oxygen,

$R_3$ cannot be equal to a propylene chain which is possibly substituted by hydroxyl, alkoxy or benzyloxy, and whereby for the case in which the group $R_1—X—R_2$ represents an alkyl chain with 1-18 carbon atoms substituted by halogen or phenyl and Y and Z signify oxygen,

$R_3$ cannot be equal to a propylene or 2-hydroxypropylene chain ;
as well as of their pharmacologically acceptable salts, characterised in that a compound of the general formula II

$$R_1—X—R_2—Y—R_3—ZH \quad (II),$$

is either

a) reacted with a compound of the general formula III or IIIa

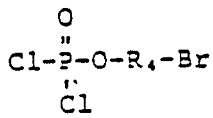 (III) 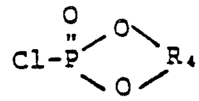 (IIIa)

in the presence of an acid-binding agent, the reaction product is selectively hydrolysed in the case of use of a compound of the formula III and the remaining bromine atom exchanged for a possibly alkylated ammonium group, in the case of use of a compound of the formula IIIa is treated directly with possibly alkylated ammonia, or

b) converted into a compound of the general formula IV

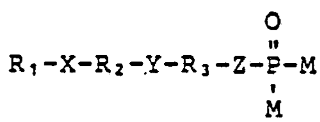 (IV)

in which M signifies hydroxyl, chlorine, bromine or alkylmercapto, and this reacted with a compound of the general formula V

$$HO—R_4—T \quad (V)$$

in which T represents chlorine or bromine or the group $N(R_5)_3^+Hal^-$, whereby $R_5$ has the above-given meaning and $Hal^-$ is to be chloride, bromide or iodide, and for the case in which T is chlorine or bromine, the intermediate product obtained is quaternised with an amine $N(R_5)_3$, or

c) reacted with a compound of the general formula VI

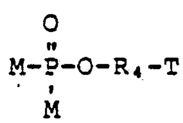 (VI)

in the presence of an acid- or water-binding agent, the compounds obtained converted, if desired, into the inner salt, possibly oxidised to the sulphones or sulphoxides, possibly quaternised and, if desired, compounds of the formula I converted into pharmacologically acceptable salts.

2. Process for the preparation of compounds of the formula Ia

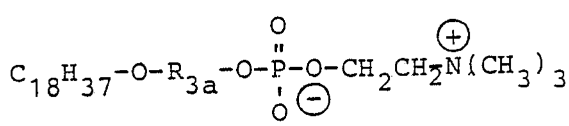 (Ia)

in which

$R_{3a}$ = 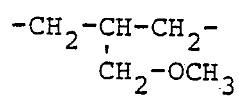 ; 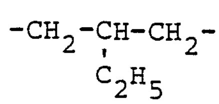 ; 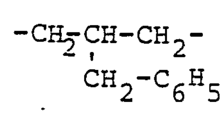 ;

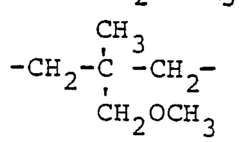 ; 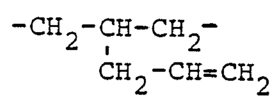

as well as of their pharmacologically acceptable salts according to claim 1, whereby the group $R_1—X—R_2 = C_{18}H_{37}$, $R_3 = R_{3a}$, Y = oxygen, Z = oxygen, $R_4 = —CH_2CH_2—$ and $R_5 = —CH_3$.

3. Process for the preparation of compounds of the formula Ib

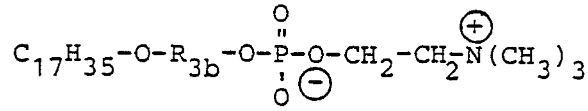

(Ib)

in which

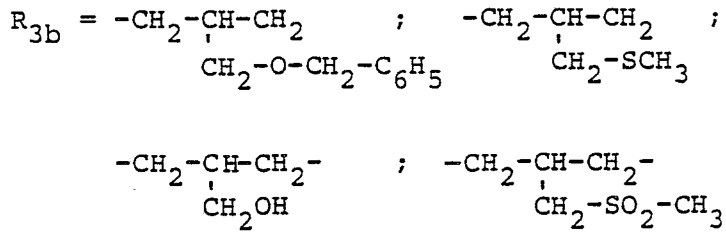

as well as of their pharmacologically acceptable salts according to claim 1, whereby the group $R_1$—X—$R_2$ = $C_{17}H_{35}$, $R_3$ = $R_{3b}$, Y = oxygen, X = oxygen, $R_4$ = —$CH_2CH_2$— and $R_5$ = —$CH_3$.

4. Process for the preparation of compounds of the formula Ic

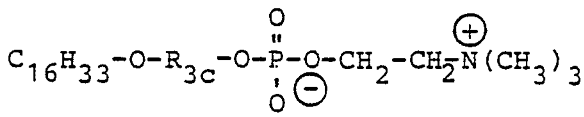

(Ic)

in which

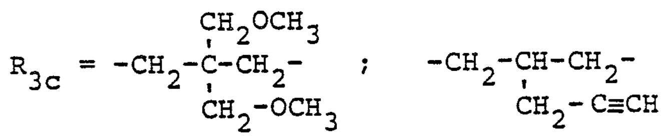

as well as of their pharmacologically acceptable salts according to claim 1, whereby the group $R_1$—X—$R_2$ = $C_{16}H_{33}$, $R_3$ = $R_{3c}$, Y = oxygen, X = oxygen, $R_4$ = —$CH_2CH_2$— and $R_5$ = —$CH_3$.

5. Process for the preparation of [2-Methyl-1-(3-tetradecyloxy-propoxy)-3-propyl]-(2-trimethylammonium-ethyl) phosphate, as well as its pharmacologically acceptable salts according to claim 1, whereby the group $R_1$—X—$R_2$ = $C_{14}H_{29}$—O—$(CH_2)_3$—, $R_3$ the group —$CH_2$—$CH_2$—$CH_2$—, Y = oxygen, Z = oxygen, $R_4$ = —$CH_2CH_2$— and $R_5$ = —$CH_3$.

**Revendications** (pour les Etats contractants : BE, CH, DE, FR, GB, IT, LI, LU, NL, SE)

1. Phospholipides de formule générale I'

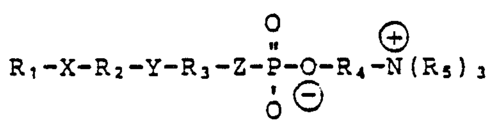

(I)

dans laquelle

X est un trait de valence, l'oxygène, le soufre, le groupe sulfonyle, le groupe sulfinyle, un reste phénylène, un reste cyclo-alkylène $C_3$-$C_8$, le groupe —CONH—, —NHCO—, —NHCONH— ou carbonyle

$R_1$ est l'hydrogène ou un reste alkyle à chaîne droite ou ramifiée, saturée ou insaturée ayant de 1 à 18 ou de 2 à 18 atomes de carbone, éventuellement substituée une ou plusieurs fois par de l'halogène, un groupe alcoxy, alkylmercapto, alcanesulfinyle, alcanesulfonyle, carbalcoxy ou le reste phényle,

$R_2$ est une chaîne alkylène droite ou ramifiée, saturée ou insaturée ayant de 1 à 18, ou de 2 à 18 atomes de carbone, substituée éventuellement une ou plusieurs fois par de l'halogène, un groupe alcoxy, alkylmercapto, alcanesulfinyle, alcanesulfonyle, carbalcoxy ou le reste phényle,

Y est l'oxygène, O—CO—O, O—CO—NH, O—CS—NH,

$R_3$ est —$CH_2$—$CH_2$—$CH_2$—, pouvant également faire partie d'un système cyclique cyclopentane, cyclohexane ou cycloheptane et substitué éventuellement une ou plusieurs fois par de l'hydroxyle, de l'halogène, un groupe alkylmercapto, alcanesulfinyle, alcanesulfonyle, le groupe nitrile, alcoxycarbonyle ou un groupe carbamoyle éventuellement substitué par des groupes alkyle, un reste cyclopentyle, cyclohexyle ou cycloheptyle, un reste phényle éventuellement substitué, ou par un groupe alcoxy à son tour éventuellement substitué par du phényle, de l'hydroxyle, un groupe alcoxy, alkylmercapto, alcanesulfinyle, alcanesulfonyle, un groupe amino éventuellement acylé, un reste alcoxycarbonyle, le reste nitrile ou par un reste carbamoyle éventuellement substitué par des groupes alkyle,

Z est l'oxygène ou le soufre,

$R_4$ est une chaîne alkylène droite ou ramifiée ayant de 2 à 5 atomes de carbone

$R_5$ est l'hydrogène ou un groupe alkyle inférieur, sous réserve que : dans le cas où le groupe $R_1$—X—$R_2$ est une chaîne alkyle droite ou ramifiée, saturée ou insaturée non substituée ayant de 1 à 18 ou de 2 à 18 atomes de carbone et Z est l'oxygène,

$R_3$ ne puisse représenter une chaîne propylène éventuellement substituée par de l'hydroxyle, de l'alcoxy ou du benzyloxy, et dans le cas où le groupe $R_1$—X—$R_2$ est une chaîne alkyle substituée par de l'halogène ou du phényle ayant de 1 à 8 atomes de carbone et Y et Z sont de l'oxygène,

$R_3$ ne puisse représenter une chaîne propylène ou 2-hydroxypropylène,

ainsi que leurs sels pharmacologiquement compatibles.

2. Composés de formule Ia

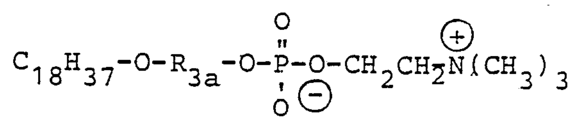

(Ia)

dans laquelle $R_{3a}$ est :

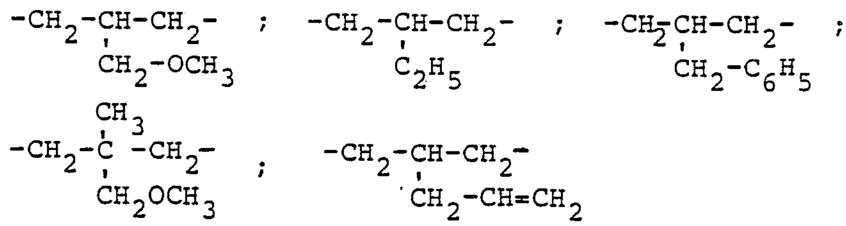

ainsi que leurs sels pharmacologiquement compatibles.

3. Composés de formule Ib

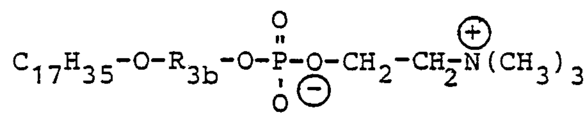

(Ib)

dans laquelle $R_{3b}$ est :

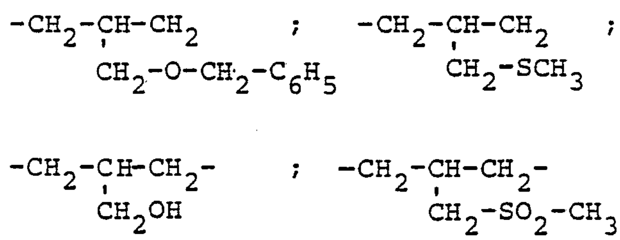

ainsi que leurs sels pharmacologiquement compatibles.

4. Composés de formule Ic

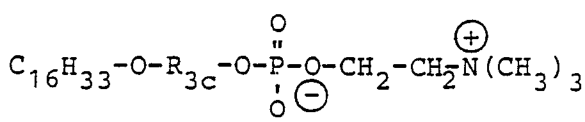

(Ic)

dans laquelle $R_{3c}$ est :

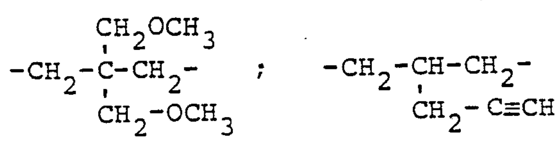

ainsi que leurs sels pharmacologiquement compatibles.

5. Le (2-triméthylammonium-éthyl) phosphate de 2-méthyl 1-(3-tétradécyloxy-propoxy) 3-propyle ainsi que ses sels pharmacologiquement compatibles.

6. Procédé pour la préparation de composés selon la revendication 1, caractérisé en ce que de façon en soi connue un composé de formule générale II

$$R_1—X—R_2—Y—R_3—ZH \quad (II),$$

est soit :

a) mis en réaction avec un composé de formule générale III ou IIIa

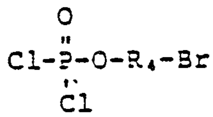 (III) 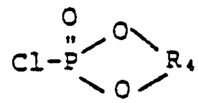 (IIIa),

en présence d'un agent liant l'acide et le produit de réaction obtenu dans le cas de la mise en œuvre d'un composé de formule III est sélectivement hydrolysé et l'atome de brome restant est échangé contre un groupe ammonium éventuellement alkylé, et dans le cas de la mise en œuvre d'un composé de formule IIIa il est traité directement par de l'ammoniac éventuellement alkylé, soit

b) transformé en un composé de formule générale IV

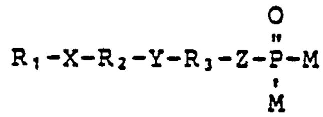 (IV)

dans laquelle M est de l'hydroxyle, du chlore, du brome ou de l'alkylmercapto, et ce composé est fait réagir avec un composé de formule générale V

$$HO—R_4—T \quad (V),$$

dans laquelle T est du chlore ou du brome ou le groupe $N(R_5)_3^+$ $hal^-$, où $R_3$ a la signification donnée ci-dessus et $hal^-$ doit être du chlorure, du bromure ou de l'iodure, et dans le cas où T est le chlore ou le brome, le produit intermédiaire ainsi obtenu est ensuite quaternisé avec une amine $N(R_5)_3$, soit

c) mis en réaction avec un composé de formule générale VI

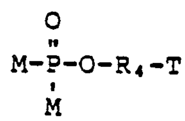 (VI)

en présence d'un agent liant l'acide ou l'eau,
et les composés obtenus sont, si on le désire, transformés en leur sel interne, éventuellement transformés par oxydation en sulfones ou sulfoxydes, éventuellement quaternisés et, si on le désire, des composés de formule I sont transformés en sels pharmacologiquement compatibles.

7. Médicament comportant un des composés des revendications 1 à 5 et des substances auxiliaires et de support habituelles.

8. Composés selon les revendications 1 à 5 pour l'utilisation dans la lutte contre le cancer.

**Revendications** (pour l'Etat contractant AT)

1. Procédé de préparation de composés de formule I

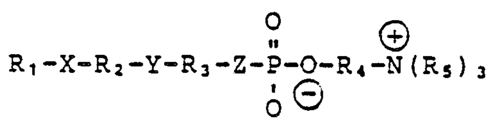 (I)

dans laquelle

X est un trait de valence, l'oxygène, le soufre, le groupe sulfonyle, le groupe sulfinyle, un reste phénylène, un reste cyclo-alkylène $C_3$-$C_8$, le groupe —CONH—, —NHCO—, —NHCONH— ou carbonyle

$R_1$ est l'hydrogène ou un reste alkyle à chaîne droite ou ramifiée, saturée ou insaturée ayant de 1 à 18 ou de 2 à 18 atomes de carbone, éventuellement substituée une ou plusieurs fois par de l'halogène, un groupe alcoxy, alkylmercapto, alcanesulfinyle, alcanesulfonyle, carbalcoxy ou le reste phényle,

$R_2$ est une chaîne alkylène droite ou ramifiée, saturée ou insaturée ayant de 1 à 18, ou de 2 à 18 atomes de carbone, substituée éventuellement une ou plusieurs fois par de l'halogène, un groupe alcoxy, alkylmercapto, alcanesulfinyle, alcanesulfonyle, carbalcoxy ou le reste phényle,

Y est l'oxygène, O—CO—O, O—CO—NH, O—CS—NH,

$R_3$ est $—CH_2—CH_2—CH_2—$,

pouvant également faire partie d'un système cyclique cyclopentane, cyclohexane ou cycloheptane et substitué éventuellement une ou plusieurs fois par de l'hydroxyle, de l'halogène, un groupe alkylmercapto, alcanesulfinyle, alcanesulfonyle, le groupe nitrile, alcoxycarbonyle ou un groupe carbamoyle éventuellement substitué par des groupes alkyle, un reste cyclopentyle, cyclohexyle ou cycloheptyle, un reste phényle éventuellement substitué, ou par un groupe alcoxy à son tour éventuellement substitué par du phényle, de l'hydroxyle, un groupe alcoxy, alkylmercapto, alcanesulfinyle, alcanesulfonyle, un groupe amino éventuellement acylé, un reste alcoxycarbonyle, le reste nitrile ou par un reste carbamoyle éventuellement substitué par des groupes alkyle,

Z est l'oxygène ou le soufre,

$R_4$ est une chaîne alkylène droite ou ramifiée ayant de 2 à 5 atomes de carbone

$R_5$ est l'hydrogène ou un groupe alkyle inférieur, sous réserve que : dans le cas où le groupe $R_1—X—R_2$ est une chaîne alkyle droite ou ramifiée, saturée ou insaturée non substituée ayant de 1 à 18 ou de 2 à 18 atomes de carbone et Z est l'oxygène,

$R_3$ ne puisse représenter une chaîne propylène éventuellement substituée par de l'hydroxyle, de l'alcoxy ou du benzyloxy, et dans le cas où le groupe $R_1—X—R_2$ est une chaîne alkyle substituée par de l'halogène ou du phényle ayant de 1 à 18 atomes de carbone et Y et Z sont de l'oxygène,

$R_3$ ne puisse représenter une chaîne propylène ou 2-hydroxypropylène,

ainsi que leurs sels pharmacologiquement compatibles, caractérisé en ce qu'un composé de formule générale II

$$R_1—X—R_2—Y—R_3—ZH \quad (II),$$

est soit :

a) mis en réaction avec un composé de formule générale III ou IIIa

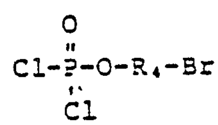 (III) 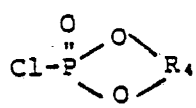 (IIIa),

en présence d'un agent liant l'acide et le produit de réaction obtenu dans le cas de la mise en œuvre d'un composé de formule III est sélectivement hydrolysé et l'atome de brome restant est échangé contre un groupe ammonium éventuellement alkylé, et dans le cas de la mise en œuvre d'un composé de formule IIIa il est traité directement par de l'ammoniac éventuellement alkylé, soit

b) transformé en un composé de formule générale IV

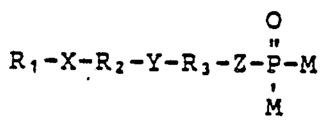 (IV)

dans laquelle M est de l'hydroxyle, du chlore, du brome ou de l'alkylmercapto, et ce composé est fait réagir avec un composé de formule générale V

$$HO—R_4—T \quad (V),$$

dans laquelle T est du chlore ou du brome ou le groupe $N(R_5)_3^+$ $hal^-$, où $R_3$ a la signification donnée ci-dessus et $hal^-$ doit être du chlorure, du bromure ou de l'iodure, et dans le cas où T est le chlore ou le brome, le produit intermédiaire ainsi obtenu est ensuite quaternisé avec une amine $N(R_5)_3$, soit

c) mis en réaction avec un composé de formule générale VI

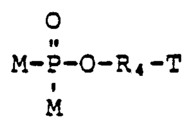 (VI)

en présence d'un agent liant l'acide ou l'eau.

2. Procédé pour la préparation de composés de formule Ia

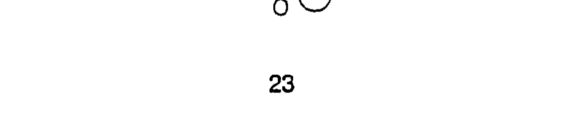 (Ia)

dans laquelle $R_{3a}$ est :

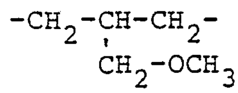 ; 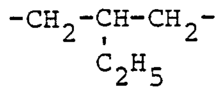 ; 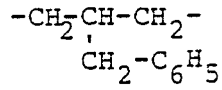 ;

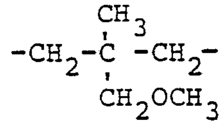 ; 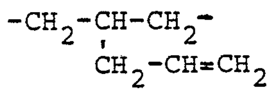

ainsi que leurs sels pharmacologiquement compatibles, selon la revendication 1, dans laquelle le groupe $R_1—X—R_2$ est $C_{18}H_{37}$, $R_3$ est $R_{3a}$, Y est l'oxygène, Z est l'oxygène, $R_4$ est $—CH_2CH_2—$ et $R_5$ est $—CH_3$.

3. Procédé pour la préparation de composés de formule Ib

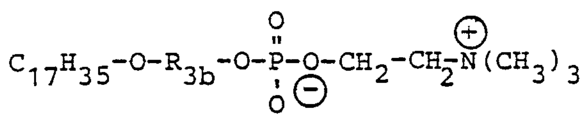 (Ib)

dans laquelle $R_{3b}$ est :

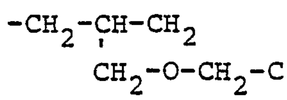 ; 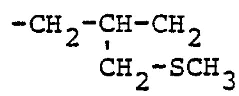 ;

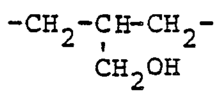 ; 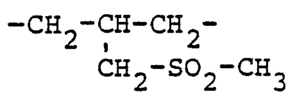

ainsi que leurs sels pharmacologiquement compatibles, selon la revendication 1 dans laquelle le groupe $R_1—X—R_2$ est $C_{17}H_{35}$, $R_3$ est $R_{3b}$, Y est l'oxygène, X est l'oxygène, $R_4$ est $—CH_2CH_2—$ et $R_5$ est $—CH_3$.

4. Procédé pour la préparation de composés de formule Ic

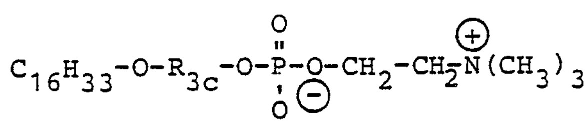 (Ic)

dans laquelle $R_{3c}$ est :

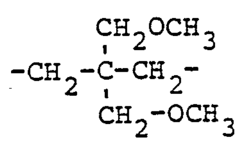 ; 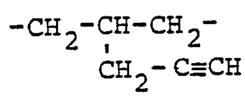

ainsi que leurs sels pharmacologiquement compatibles selon la revendication 1 dans laquelle le groupe $R_1—X—R_2$ est $C_{16}H_{33}$, $R_3$ est $R_{3c}$, Y est l'oxygène, X est l'oxygène, $R_4$ est $—CH_2CH_2—$ et $R_5$ est $—CH_3$.

5. Procédé pour la préparation du (2-triméthylammonium-éthyl) phosphate de 2-méthyl 1-(3-tétradécyloxypropoxy) 3-propyle ainsi que ses sels pharmacologiquement compatibles selon la revendication 1 dans laquelle le groupe $R_1—X—R_2$ est $C_{14}H_{29}—O—(CH_2)_3—$, $R_3$ est le groupe $—CH_2—CH_2—CH_2—$, Y est l'oxygène, Z est l'oxygène, $R_4$ est $—CH_2CH_2—$ et $R_5$ est $—CH_3$.